# FASCICULE DE BREVET EUROPEEN

(11) **EP 2 446 276 B1**
(45) Date de publication et mention de la délivrance du brevet: **20.08.2014**
(21) Numéro de dépôt: 10745670.9
(22) Date de dépôt: 25.06.2010
(51) Int. Cl.: G01N 33/92, G01N 33/52, G01N 33/574, G01N 33/58

(54) **MÉTHODES POUR DÉTERMINER L'ÉTAT ONCOGÉNIQUE D'UNE CELLULE, LEURS APPLICATIONS, ET MÉTHODES POUR TRAITER LE CANCER**
VERFAHREN ZUR BESTIMMUNG DES ONKOGENEN ZUSTANDS EINER ZELLE, ANWENDUNGEN DAVON UND VERFAHREN ZUR KREBSBEHANDLUNG
METHODS FOR DETERMINING THE ONCOGENIC CONDITION OF CELL, USES THEREOF, AND METHODS FOR TREATING CANCER

(30) Priorité: 25.06.2009 FR 0903101
(43) Date de publication de la demande: 02.05.2012
(73) Titulaire: INSERM (Institut National de la Santé et de la Recherche Médicale), 75013 Paris (FR); Affichem, 31400 Toulouse (FR)
(72) Inventeur: DE MEDINA, Philippe, 31170 Colomiers (FR); PAILLASSE, Michaël, 31300 Toulouse (FR); POIROT, Marc, 31240 L'Union (FR); SILVENTE-POIROT, Sandrine, 31240 L'Union (FR)
(74) Mandataire: Cabinet Plasseraud
(86) Numéro de dépôt international: PCT/FR2010/051320
(87) Numéro de publication internationale: WO 2010/149941

(56) Documents cités:
- WO-A1-93/15227
- SANTEN RICHARD J ET AL.: "Potential role of ultra-sensitive estradiol assays in estimating the risk of breast cancer and fractures." STEROIDS LNKD- PUBMED:18614192, vol. 73, no. 13, 12 décembre 2008 (2008-12-12), pages 1318-1321, XP25584477 ISSN: 0039-128X DOI: 10.1016/j.steroids.2008.05.008
- MATSUSHIMA YOUKO ET AL.: "Enhancement of Human Papillomavirus Type 18 Gene Expression in HeLa Cells by 12-O-Tetradecanoylphorbol-13-acetate, 3-beta,5-alpha-Dihydroxycholestan-6-one, and Cholesterol" BIOLOGICAL AND PHARMACEUTICAL BULLETIN, vol. 17, no. 9, septembre 1994 (1994-09), pages 1292-1295, XP9131671 ISSN: 0918-6158
- AZUMA AKIHIKO ET AL.: "Photoaffinity labeling of tumor promoter-binding protein (CN-TPBP) and preparation of affinity sorbent gels" BIOLOGICAL AND PHARMACEUTICAL BULLETIN, vol. 20, no. 1, 1997, pages 6-9, XP9131669 ISSN: 0918-6158
- YAMAGUCHI MITSUHIRO ET AL.: "3beta,5alpha-dihydroxycholestan-6-one exists in human blood" BIOLOGICAL AND PHARMACEUTICAL BULLETIN, vol. 20, no. 9, septembre 1997 (1997-09), pages 1044-1046, XP9131626 ISSN: 0918-6158 cité dans la demande
- SRIVASTAVA S ET AL.: "Translational Research Working Group developmental pathway for biospecimen-based assessment modalities" CLINICAL CANCER RESEARCH, vol. 14, no. 18, 15 septembre 2008 (2008-09-15), pages 5672-5677, XP9139990 ISSN: 1078-0432 DOI: 10.1158/1078-0432.CCR-08-1267
- SUMI SHOICHIRO ET AL.: "Lovastatin inhibits pancreatic cancer growth regardless of RAS mutation" PANCREAS, vol. 9, no. 5, septembre 1994 (1994-09), pages 657-661, XP9139970 US ISSN: 0885-3177
- BOK R A ET AL.: "The treatment of advanced prostate cancer with ketoconazole: safety issues." DRUG SAFETY : AN INTERNATIONAL JOURNAL OF MEDICAL TOXICOLOGY AND DRUG EXPERIENCE LNKD- PUBMED:10348095, vol. 20, no. 5, mai 1999 (1999-05), pages 451-458, XP9139971 ISSN: 0114-5916 DOI: 10.2165/00002018-199920050-00005
- BRYCE C J: "Tamoxifen in early breast cancer" THE LANCET, vol. 352, no. 9125, 1 août 1998 (1998-08-01), page 403, XP009139995 LANCET LIMITED. LONDON, GB ISSN: 0140-6736 DOI: 10.1016/S0140-6736(05)60500-4
- MONTZ F J ET AL.: "Intrauterine progesterone treatment of early endometrial cancer" AMERICAN JOURNAL OF OBSTETRICS & GYNECOLOGY, vol. 186, no. 4, avril 2002 (2002-04), pages 651-657, XP008026788 MOSBY, ST LOUIS, MO, US ISSN: 0002-9378 DOI: 10.1067/MOB.2002.122130

### SEQUENCE LISTING

<110> INSERM (Institut National de la Santé et de la Recherche Médicale) AFFICHEM
<120> Méthodes pour déterminer l'état oncogénique d'une cellule, leurs applications, et méthodes pour traiter le cancer
<130> BCT100197
<160> 4
<170> PatentIn version 3.3
<210> 1
   <211> 21
   <212> DNA
   <213> Artificial
<220>
   <223> Primer
<400> 1
   aaaccaaacc acaagacaga c 21
<210> 2
   <211> 18
   <212> DNA
   <213> Artificial
<220>
   <223> Primer
<400> 2
   gctgaaggca tctcggag 18
<210> 3
   <211> 21
   <212> DNA
   <213> Artificial
<220>
   <223> Primer
<400> 3
   ctatggtgag ccgtgattgt g 21
<210> 4
   <211> 21
   <212> DNA
   <213> Artificial
<220>
   <223> Primer
<400> 4
   tctgtgtcat cctcctgtgt c 21

## Description

### Domaine de l'invention

L'invention concerne des méthodes pour déterminer l'état oncogénique d'une cellule et leurs applications. L'invention concerne également des inhibiteurs d'OCDO pour une utilisation dans des méthodes de traitement de cancers.

### Introduction

Le diagnostic d'un cancer repose sur différents éléments comme notamment l'auscultation et les examens médicaux (endoscopie, fibroscopie, radioscopie, etc.), qui permettent au médecin de constater les signes visibles ou palpables de la maladie, mais également les analyses de sang et d'urine qui permettent de connaître le nombre de globules rouges, de globules blancs et de plaquettes, le taux d'hémoglobine, le taux de créatinine, ou encore sur la détection de la présence de marqueurs spécifiques du cancer dans des échantillons prélevés chez le patient. De nombreux marqueurs du cancer ont été décrits dans la littérature. Ceux-ci sont généralement spécifiques de certains types de cancers en particulier, voire de sous-populations de patients. Par ailleurs, les marqueurs décrits dans la littérature ne permettent généralement pas de diagnostiquer le cancer de manière certaine, et les taux de faux-négatifs ou faux-positifs sont encore importants. Enfin, certains marqueurs ne sont détectables qu'à un stade tardif du développement du cancer, ce qui compromet au moins en partie la réussite du traitement.

Il existe donc un besoin pour de nouveaux marqueurs du cancer, permettant de diagnostiquer n'importe quel type de cancer, et cela à un stade précoce et de manière sûre et reproductible.

Azuma A et al. Photoaffinity labeling of tumor promoter-binding protein (CN-TPBP) and préparation of affinity sorbent gels. Biol Pharm Bull. 1997 Jan;20(1):6-9 est relatif à des méthodes de dosage de l'OCDO.

Matsushima Yet al. Enhancement of human papillomavirus type 18 gene expression in HeLa cells by 12-O-tetradecanoylphorbol-13-acetate, 3 beta,5 alpha-dihydroxycholestan-6-one, and cholesterol. Biol Pharm Bull. 1994 Sep;17(9):1292-5 est relatif au rôle de l'OCDO comme inducteur du gène viral HPV18.

### Résumé de l'invention

L'invention concerne une méthode pour détecter l'état oncogénique de cellules d'un échantillon obtenu chez un sujet, caractérisée en ce qu'elle comprend une étape où l'on détermine la quantité du composé OCDO de formule 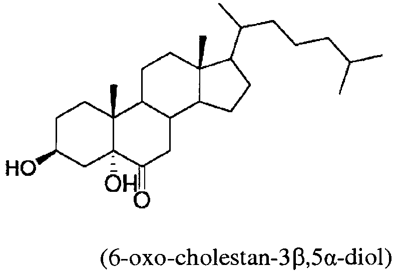 présente dans les cellules prélevées.

L'invention concerne également une méthode pour diagnostiquer un cancer chez un sujet, caractérisée en ce qu'elle comprend une étape de détection de l'état oncogénique de cellules d'un échantillon obtenu chez un sujet selon la méthode de détection de l'invention dans laquelle une quantité d'OCDO supérieure à une valeur de référence mesurée dans les cellules d'un échantillon de sujet sain est indicative d'un état oncogénique des cellules dudit échantillon.

L'invention concerne aussi l'utilisation du composé OCDO présent dans des cellules d'un échantillon obtenu chez un sujet comme marqueur de l'état oncogénique desdites cellules.

L'invention concerne également une méthode pour suivre la réponse à un traitement d'un sujet souffrant d'un cancer, ladite méthode comprenant les étapes de détection, avant et au cours du traitement, de l'état oncogénique de cellules d'un échantillon obtenu chez ledit sujet selon la méthode de détection de l'invention; un changement de l'état oncogénique du sujet au cours du traitement étant indicatif d'une réponse du sujet audit traitement.

L'invention concerne également une méthode pour évaluer l'efficacité d'un médicament pour traiter un cancer chez un sujet atteint par ledit cancer, comprenant :
(a) une étape de détection de l'état oncogénique de cellules d'un échantillon obtenu chez un sujet selon la méthode de détection de l'invention où on dose la concentration D1 d'OCDO dans un extrait liquide des cellules d'un échantillon obtenu chez ledit sujet;
(b) après un temps de traitement thérapeutique, une étape de détection de l'état oncogénique de cellules d'un échantillon obtenu chez un sujet selon la méthode de détection de l'invention où on dose la concentration D2 d'OCDO dans un extrait liquide des cellules d'un échantillon obtenu chez ledit sujet de la même manière qu'à l'étape (a);
(c) une étape de comparaison de D1 et D2 ; et
(d) si D2 < D1, on en déduit que le médicament est efficace pour traiter ledit cancer.

L'invention a aussi pour objet une méthode pour évaluer l'efficacité d'un traitement du cancer chez un sujet atteint par ledit cancer, ladite méthode comprenant les étapes de détection, avant et au cours du traitement, de l'état oncogénique de cellules d'un échantillon obtenu chez ledit sujet selon la méthode de détection de l'invention; un changement de l'état oncogénique ou du diagnostic du sujet au cours du traitement étant indicatif de l'efficacité dudit traitement pour traiter le cancer.

L'invention concerne aussi les inhibiteurs de l'OCDO pour une utilisation dans une méthode de traitement d'un cancer chez l'homme ou l'animal, ledit inhibiteur étant choisi parmi les inhibiteurs de l'activité cholestérol-époxy hydrolase (ChEH) choisis dans le groupe consistant en le Ro 48-8071 et l'ibogaïne.

### Définitions

On entend par « matériau biologique » ou « échantillon » au sens de l'invention, un tissu biologique, une préparation ou un extrait issu de tissu biologique, liquide ou solide ; le matériau peut aussi être un mélange d'au moins deux matériaux tels que ci-dessus définis. Un tel échantillon ou matériau biologique peut donc être, notamment, soit préparé à partir de tissus, d'organes, de selles ou de liquides biologiques d'un homme ou d'un mammifère, soit obtenu à partir de cultures cellulaires « in vitro » ; un tel échantillon ou matériau biologique peut aussi être du sang, du sérum, du plasma, de l'urine, du liquide céphalorachidien, du liquide synovial, du liquide péritonéal, du liquide pleural, du liquide séminal ou du liquide ascitique. Typiquement un échantillon ou matériau biologique selon l'invention est une biopsie d'un tissu cancéreux ou suspecté d'être cancéreux.

Par « sujet » au sens de l'invention, on entend un mammifère, humain ou animal.

Par « état oncogénique » d'une cellule on entend un état dédifférencié dans lequel le programme de prolifération de la cellule a été modifié de telle sorte que celle-ci prolifère de manière non contrôlée, pouvant conduire à la formation de tumeurs malignes, invasives et/ou métastasiques.

Les termes « traitement » et « traiter » se réfèrent à tout acte tendant à améliorer l'état de santé d'un sujet, comme la thérapie, la prévention, la prophylaxie, ou le ralentissement de la maladie. Dans certains modes de réalisation, ces termes se réfèrent à l'amélioration ou l'éradication d'une maladie ou de symptômes associés à cette maladie. Dans d'autres modes de réalisation, ces termes se réfèrent à la diminution de la progression ou de la malignité de la maladie.

Par « quantité thérapeutiquement efficace », on entend une quantité suffisante pour traiter le sujet.

Par « cancer » on entend tout type de maladie dans laquelle certaines cellules du corps humain ou animal se divisent d'une manière incontrôlée. Les cancers sont typiquement choisis parmi les carcinomes, les sarcomes et les cancers hématopoïétiques. Plus particulièrement le cancer selon l'invention est un cancer du sein, un cancer du poumon, un mélanome, un cancer du côlon, un cancer du rectum, un cancer du pancréas, un myélome multiple, une leucémie, un lymphome, un sarcome de Kaposi, un cancer des testicules, un cancer de la prostate, un cancer de l'utérus, un gliome, un neuroblastome, un ostéosarcome, un carcinome embryonnaire, un carcinome médullaire de la thyroïde.

### Description détaillée de l'invention

La présente invention est relative à l'identification d'un marqueur, l'OCDO, qui apparaît dans des cellules tumorales de manière précoce et qui présente des propriétés mitogènes et de stimulation de l'invasivité tumorale. L'invention est basée sur la détection de ce marqueur et son dosage dans des échantillons obtenus chez les sujets suspectés d'être atteints ou atteints d'un cancer. L'invention concerne en outre des molécules inhibitrices de l'OCDO pour leur utilisation dans des méthodes de traitement des cancers.

Le cholestérol et les produits d'oxydation du cholestérol sont soupçonnés depuis longtemps de présenter des propriétés carcinogènes chez l'homme (Fritz Bischof, Advances in Lipid Research, vol 7, p. 165-244, 1969). Ces effets ont été initialement observés sur un nombre limité d'espèces de rongeurs et ont conduit à des observations contradictoires qui ont entraîné un désintéressement de la communauté scientifique sur ce sujet (Leland L Smith et al., Free Radical Biology and Medecine, Vol 7, p. 285-332, 1989). Certains produits d'oxydation du cholestérol sont connus pour apparaître lors du catabolisme du cholestérol et de la production des hormones stéroïdes. Un certain nombre d'oxystérols jouent un rôle physiologique clé dans le système immunitaire, le système nerveux et le système cardiovasculaire. Les cibles connues des oxystérols sont des facteurs de transcription ligands-dépendant, dont les plus connus sont les récepteurs LXR (liver-X-receptor). Les oxysterols modulent le transport intracellulaire du cholestérol, la formation de micro-domaines lipidiques, l'activation des voies de HedgeHog impliquées dans la morphogenèse lors du développement embryonnaire ; ils ont aussi des propriétés antagonistes sur les récepteurs des hydrocarbures aromatiques.

On a indiqué que certains oxystérols modulent l'activité d'enzymes impliqués dans le métabolisme du cholestérol et, notamment, dans la biosynthèse de mévalonate (HMG-CoA-réductase), l'esterification du cholestérol, l'hydrolyse des époxydes de cholestérol (voir Schoepfer G. Jr. : Physiological Reviews, Vol 80, N°1, p. 361-554, 2000). On a noté aussi que, selon la nature du groupement oxygéné (alcool, carbonyle, hydroperoxyde, peroxyde ou époxyde), sa position sur le squelette hydrocarboné du cholestérol, son orientation dans l'espace et le nombre de groupements oxygénés sur ledit squelette hydrocarboné, un produit d'oxydation du cholestérol peut présenter des propriétés de reconnaissance spécifiques de différentes cibles biologiques (membranes, enzymes, récepteurs).

Selon l'invention, on a constaté que le cholestane-3ß,5α-diol-6-one (OCDO) était présent dans des lignées tumorales d'origine tissulaire variées. On a caractérisé les propriétés de cette molécule dans la mitogenèse tumorale et, in vitro, dans l'invasivité sur des cellules et sur des tumeurs implantées chez le rongeur. On a, de plus, montré que cette molécule entraîne, dans les cellules tumorales, une stimulation de l'expression de cytokines immunosuppressives alors qu'elle diminue l'expression de cytokines immunostimulantes, ce qui est en accord avec une immuno-suppression au voisinage de la tumeur favorisant son développement et son invasivité.

On sait que la cholestérol-époxyde hydrolase (ChEH) est une enzyme responsable de l'hydrolyse de l'époxyde de cholestérol (CE) en cholestane triol (CT) selon la réaction suivante : 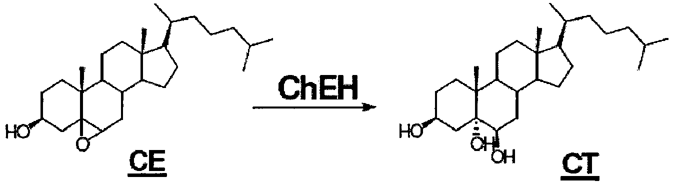 (voir de Médina et al, Faseb J. 19(4) : A285 - A285, Part 1 Suppl. S, 04 Mars 2005).

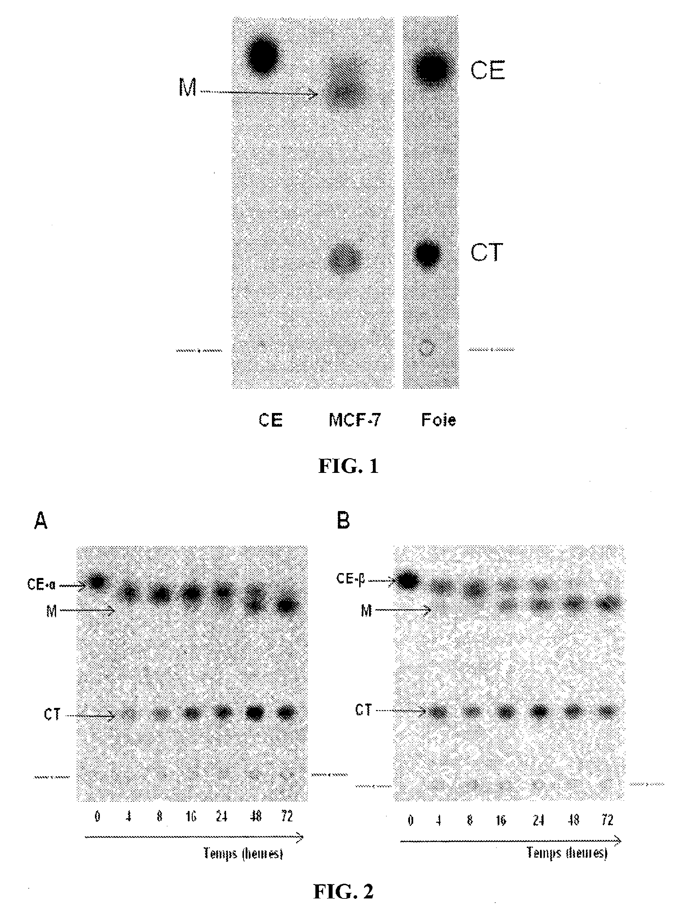

Dans un premier temps, les demanderesses ont comparé l'activité de la ChEH pour des extraits cellulaires obtenus à partir de cellules de tissus normaux et à partir de cellules tumorales : l'activité de la ChEH peut être observée en séparant par chromatographie sur couche mince, le substrat CE par rapport au produit CT de la ChEH. La visualisation des taches correspondant aux composés CE et CT a été réalisée par radiomarquage à ¹⁴C. Le détail de l'expérimentation est fourni à l'essai 1 détaillé plus loin et le résultat est représenté sur la figure 1.

Les demanderesses ont constaté, que les cellules normales permettaient de visualiser deux taches correspondant aux composés CE et CT, mais qu'en revanche, de façon surprenante, les cellules tumorales faisaient apparaître trois taches, à savoir l'une correspondant au cholestane triol (CT), la deuxième correspondant à l'époxyde de cholestérol (CE) (faible par rapport à la tache correspondante relative aux cellules normales), et une troisième tache proche de celle du composé CE mais néanmoins parfaitement distincte.

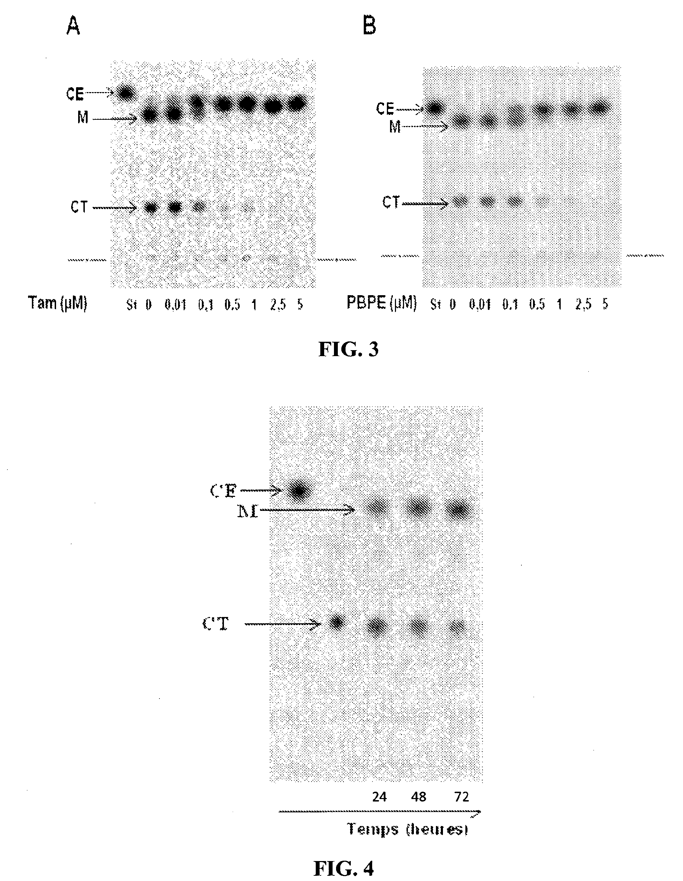

On sait que le tamoxifène (tam), utilisé de façon connue comme agent anticancéreux réducteur de tumeurs pour le traitement et la prévention des cancers du sein, est un inhibiteur de la ChEH et qu'il en est de même pour le PBPE (N,N-pyrrolidino-[(4-benzyl-phenoxy]-éthanamine) qui est un agent anti-prolifératif connu (voir à cet égard la référence de publication donnée dans l'essai 2 de la présente demande). Ni cette « troisième tache », que les demanderesses ont attribuée à un métabolite M, ni la tache CT n'existent, si les cellules tumorales sont traitées au tamoxifène. Sachant que le tamoxifène inhibe la ChEH, on a recherché, dans un deuxième temps, si l'inhibition de la ChEH pouvait être à l'origine de l'apparition de la « troisième tache » sur la plaque de chromatographie des cellules tumorales traitées. Les détails de cette expérience sont fournis à l'essai 2 décrit plus loin et les résultats sont représentés sur les figures 3A et 3B. Pour ce faire, on a effectué, sur des extraits de cellules tumorales traitées respectivement au tamoxifène ou au PBPE, une inhibition de l'activité ChEH, l'incubation des cellules par l'inhibiteur étant maintenue pendant trois jours, avec des doses croissantes d'inhibiteur pour les essais successifs. Et on a constaté, sur les chromatographies en couche mince que, lorsque la dose d'inhibiteur augmente, le composé CT disparaît en même temps que la « troisième tache » voisine de celle du composé CE. Selon l'invention, la demanderesse en a déduit que le métabolite M relatif à la « troisième tache » était probablement un dérivé du composé CT.

Dans un troisième temps, les demanderesses ont complété cette expérimentation en établissant que la formation du métabolite M correspondant à la « troisième tache » s'effectuait progressivement, après une incubation de plus de 24 heures avec l'inhibiteur tamoxifène de la ChEH, au détriment de la formation du composé CE ; et ce phénomène se produit avec les deux isomères α et ß du composé CE. Ce complément de démonstration est détaillé à l'essai 3 et représenté sur les figures 2A et 2B. Les demanderesses en ont donc conclu, selon l'invention, que le produit relatif à la « troisième tache », est un produit de transformation du composé CT, qui a un comportement chromatographique proche de celui du composé CE mais est retenu de façon plus importante par le support de chromatographie, ce qui indique que le métabolite M associé à la « troisième tache » est un composé ayant une polarité intermédiaire entre celles des composés CT et CE et ayant une structure voisine de celle des deux molécules CE et CT.

L'essai 4 détaillé plus loin, fournit toutes les précisions de l'étude menée pour confirmer que le métabolite M de la « troisième tache » provient d'une transformation du composé CT ; le résultat de cet essai est représenté sur la figure 4.

Enfin, l'essai 5 décrit la méthode, qui a permis l'identification chimique du produit correspondant à cette « troisième tache ». Les demanderesses ont donc établi, selon l'invention, que cette « troisième tache », qui apparaît uniquement avec les cellules tumorales testées, est due au 6-oxo-cholestan-3ß,5α-diol (OCDO) correspondant à la formule ci-dessous :

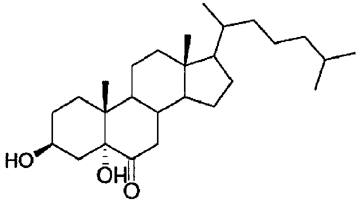

Le produit OCDO n'est pas un produit nouveau : il a été décrit comme un produit d'oxydation du Cholestane-3ß,5α,6ß-triol (CT) par la N-bromosuccinimide (Fieser L. F. et ; Rajagopalan S. : Selective oxydation with N-bromosuccinimide. II. Cholestane-3ß,5α,6ß-triol, J Am Chem Soc, 71, p. 3938-41, 1949). La synthèse chimique de l'OCDO avait, d'ailleurs déjà été décrite en 1908 (Robert Howson Pickard et al., J. Chem. Soc. Trans. vol 93, p. 1678-1687, 1908).

L'existence de cette molécule comme métabolite issu de la transformation du cholestane-3ß,5α,6ß-triol a été rapportée en 1971 : on a indiqué que l'OCDO pouvait être retrouvé dans les féces de rat gavé avec du cholestane-3ß,5α,6ß-triol (Roscoe HG, Fahrenbach MJ, J Lipid Res, vol 12, p. 17-23, 1971). On a aussi indiqué que l'OCDO pouvait être trouvé dans le sérum de bovin et le sang humain à une concentration comprise entre 10 et 100 nM (Yamaguchi M. et al, Biol. Pharm. Bull. 20(9), p. 1044-46, 1997).

On va donner ci-après la procédure expérimentale détaillée mise en oeuvre pour les essais 1 à 5, qui ont permis d'aboutir à l'identification du marqueur OCDO.

Pour les essais 1 à 5 et dans certains des exemples donnés plus loin dans cette demande de brevet, on a utilisé des cellules tumorales MCF7, qui proviennent de l'American Tissue Culture Collection (ATCC). Ces cellules sont cultivées dans du milieu RPMI 1640 supplémenté par 2g/litre de carbonate de sodium aqueux, 1,2mM de glutamine (pH 7,4 à 23°C) et 5% de sérum bovin foetal (Gibco), à 37°C sous 5% CO2, et 2,5 ml d'antibiotiques (pénicilline/streptomycine) par litre de milieu. On désire étudier l'activité de la ChEH dans des cellules de souris par chromatographie sur couche mince de silice. Les composés que l'on désire visualiser sont les composés CE et CT précédemment définis. Afin de visualiser les taches correspondant à CE et CT sur une plaque de chromatographie, on a synthétisé les composés CE et CT marqués au ¹⁴C.

### a) Synthèse du 5,6-ßépoxycholestan-3ß-ol[¹⁴C] et du 5,6-αépoxycholestan-3ß-ol[¹⁴C].

On dissout 0,35 µmol de cholestérol [¹⁴C] (58 mCi/mmol) dans 200 µl de dichlorométhane en présence de 0,56 µmol d'acide métachloroperbenzoïque. La solution est agitée à température ambiante pendant 5 heures. Le mélange réactionnel est dissout dans 1 ml de dichlorométhane, lavé avec du sulfite de sodium aqueux (10 % en poids), de l'hydrogénocarbonate de sodium (solution acqueuse à 5% en poids) et d'une solution saturée en chlorure de sodium. La phase organique est évaporée et le résidu est purifié par RP-HPLC (colonne hydrophobe Ultrasep ES C18 6µm) en condition isocratique CH₃OH/H₂O (95/5 en volume) à 0.7 ml/mn. Les isomères α et ß sont facilement séparés dans ces conditions et détectés par un détecteur de radioactivité (Berthold).

Le rendement total de la réaction est de 80 % ; le produit obtenu contient 75 % d'isomère α (CEα) et 25 % 5 d'isomère ß (CEß).

### b) Synthèse du cholestan 3ß,5α,6ß triol[¹⁴C] :

Ce composé a été synthétisé à partir 5-6βépoxycholestan-3ßol[¹⁴C] comme décrit dans la littérature (Pulfer MK and Murphy RC, Formation of biologically active oxysterols during ozonolysis of cholesterol present in lung surfactant, J Biol Chem, vol 279(25) p. 26331-26338, 2004). Le CEβ[¹⁴C] préparé sous a) ci-dessus (58 mCi/mmol) est dissout dans 1 ml d'un mélange tétrahydrofurane/H2O/acétone (v/v/v, 4 :1 : 0.5). 125µl d'acide perchlorique sont ajoutés au mélange réactionnel, qui est agité pendant 4 heures à température ambiante. Le mélange réactionnel est dilué dans 1 ml de dichlorométhane puis lavé à l'hydrogénocarbonate de sodium (solution aqueuse à 5 % en poids) et à l'eau. Le résidu est purifié par HPLC sur colonne hydrophobe (Ultrasep ES C18 6 µm) en condition isocratique CH₃OH/H₂O (95/5 en volume) à un débit de 0.7 ml/mn. On obtient le CT[¹⁴C] avec un rendement de 62%.

Pour être utilisées dans les essais 1 à 5 qui suivent, les cellules sont ensemencées dans des plaques 6 puits à une densité de 80000 cellules dans un volume de 2 ml. Trente six heures après ensemencement, les cellules sont traitées pendant 15 minutes soit par du solvant véhicule (éthanol à 1‰ dans un tampon PBS), soit par les composés que l'on désire tester ; cette incubation est donc, selon les besoins de l'essai, effectuée avec les composés CEα[¹⁴C] (0.6 µM, 15 µCi/µmol) et CEß[¹⁴C] (0,6 µM, 15µCi/µmol) ou CT [¹⁴C] (1 µM, 15 µCi/µmol), la proportion de solvant ne dépasse pas 2 ‰ par rapport au volume du milieu de culture. Après le temps d'incubation que l'on désire pour l'essai, le milieu est collecté, les cellules sont lavées par du PBS (tampon phosphate) froid (2 ml par puit) poolé avec le milieu. Les cellules sont alors grattées dans du PBS froid (1 ml pour 3 puits) ; les puits sont à nouveau rincés par du PBS froid (1 ml pour 3 puits). La suspension cellulaire obtenue est centrifugée à 1000 t/mn pendant 5 mn à 4°C. Le culot cellulaire et le milieu sont extraits par la méthode de Folch modifiée (telle que publiée par Ways P. et al., J lipid Res, 5(3) : 318 (1964)). Dans toute la suite de cette description, le solvant véhicule utilisé est le même que celui-ci-dessus défini.

Les radioactivités aqueuse et organique sont comptées. Les phases organiques sont ramenées à sec sous argon. Le résidu est resuspendu dans 60 µl d'éthanol puis déposé, à raison de 20 µl par piste, sur les plaques de silice sur verre (LK-6-DFWhatman 20x20), que l'on utilise dans les différents essais (ces plaques ayant été préalablement chauffées à 100°C pendant 1 heure). Le solvant de migration utilisé est l'acétate d'éthyle. Les plaques de chromatographie sont placées en contact avec une plaque « Phosphor-screen » dans une cassette pendant une nuit. Le « Phosphor-screen » est révélé avec un « Phosphor Imager » de type Storm. Pour évaluer le caractère plus ou moins dense des taches obtenues sur les plaques, on quantifie la radioactivité par densitométrie avec le programme informatique « Imagequant »^{(™)}.

### Essai 1

Dans cet essai 1, on a mis en évidence l'activité de la ChEH dans des cellules de souris saines et dans des cellules de souris tumorales MCF7. On procède par chromatographie en couche mince selon les techniques qui viennent d'être exposées. Les résultats sont présentés sur la figure 1.

Sur cette figure, on voit que tous les dépôts ont été faits au même niveau repéré par un trait mixte en bas des pistes.

Sur la piste de gauche, on a déposé le composé CE[¹⁴C] préalablement préparé comme indiqué ci-dessus (0,6µM, 15µCi/(µmol). Les pistes médiane et de droite correspondent à des extraits de cellules préalablement incubées dans CE[¹⁴C] selon la technique précédemment exposée.

L'extrait cellulaire déposé en bas de la piste de droite, est un extrait correspondant à des hépatocytes normaux prélevés sur des souris adultes C57/B16 de 20-25 g (fournies par Charles Rivers). Les hépatocytes ont été isolés par perfusion de collagénase selon le protocole de Davis (Davis RA et al, J. Biol Chem, 1979, Vol. 254, N° 6, p. 2010-2016) et mis en culture dans des boites de Pétri de 6 cm de diamètre recouverte de collagène, à une densité de 2 millions de cellules par boite, dans du milieu nutritif « Dulbecco modified eagle médium » (DMEM) contenant 10% de sérum de veau foetal, de l'insuline (0.5 U/ml) et des antibiotiques (50 unités/ml) (un mélange pénicilline et streptomycine). Les boites sont maintenues à 37°C dans une étuve humide avec 5% de CO2. Après adhérence des cellules, le milieu nutritif est remplacé par du milieu neuf après avoir effectué un lavage des cellules avec du tampon PBS de façon à éliminer les débris cellulaires. Les cellules sont utilisées dès le lendemain pour le test.

On voit, sur la piste de droite de la figure 1, que le composé CT apparaît en premier et que le composé CE se trouve sensiblement au niveau de celui qui a été déposé directement sur la piste de gauche. Le composé CT formé provient nécessairement du composé CE transformé par l'hydrolase ChEH, puisque l'un et l'autre apparaissent sur la chromatographie, alors qu'initialement, seul le composé marqué CE était déposé et susceptible d'apparaître.

En revanche, sur la piste médiane, on a déposé des extraits de cellules de souris tumorales MCF7; cet extrait cellulaire est préparé à partir de cellules MCF7 cultivées de la même façon que l'extrait d'hépatocytes à partir des cellules de souris C57/B16. On constate une tache correspondant au composé CT puis deux taches, proches l'une de l'autre, correspondant l'une au composé CE de la piste de droite et l'autre à un métabolite M non identifié ; de plus, cette « troisième tache » est plus intense que celle correspondant au composé CE.

On en a donc déduit que, dans les cellules tumorales, une partie du composé CE avait été transformé en un métabolite M ayant un comportement chromatographique proche de celui du composé CE et ayant une polarité intermédiaire entre celles des composés CE et CT.

### Essai 2

On a recherché si l'apparition de la « troisième tache » sur la plaque de chromatographie de l'essai 1, était ou non affectée par une inhibition de la ChEH, qui, dans la cellule, donne naissance au composé CT à partir du composé CE. On sait que la ChEH est inhibée par le tamoxifène (Tam) et par la PBPE (FASEB Journal, Vol. 19, Issue 4, p. A285-A285, Part 1 Suppl. S). Pour cet essai, on a donc utilisé ces deux inhibiteurs.

Pour ce faire, on a déposé sur une plaque de chromatographie, du même type que celle utilisée pour l'essai 1, des extraits cellulaires de cellules tumorales MCF7 incubées dans une solution de CE (-α ou -ß) [¹⁴C] comme indiqué à l'essai 1, puis, ensuite, incubées dans une solution aqueuse d'inhibiteur de ChEH, tamoxifène ou PBPE, pendant 3 jours. Quand on utilise une incubation avec CE- α [¹⁴C], on met en oeuvre des solutions de tamoxifène à des concentrations de 1.10⁻², 1.10⁻¹, 5.10⁻¹, 1, 2,5 et 5 µM (figure 3A) ; quand on utilise une incubation avec le CEß[¹⁴C], on met en oeuvre des solutions de PBPE à des concentrations de 1.10⁻², 1.10⁻¹, 1, 5 et 10 µM (figure 3B) ; les figures 3A et 3B représentent les plaques chromatographiques obtenues dans les deux cas. On constate que, pour les fortes quantités de l'inhibiteur d'incubation, on a disparition du composé CT et, simultanément, disparition de la « troisième tache » due au métabolite M ; au contraire, pour de faibles quantités d'inhibiteur, la tache du composé CT est importante de même que la tache due au métabolite M alors que les taches dues au CE sont faibles ce qui montre que le CE a été transformé en (CT+ métabolite) ; on en conclut que l'hydrolase ChEH, quand elle est peu inhibée, laisse apparaître le CT et que, dès lors, la tache du métabolite M apparaît également.

### Essai 3

On a étudié la cinétique de l'activité de la ChEH dans des cellules MCF7 en utilisant le composé CE (-α ou -ß) marqué au [¹⁴C]. Comme dans l'essai 2, les cellules sont incubées au CE (-α ou -ß)[¹⁴C]. Les extraits sont déposés sur les plaques de chromatographie après des temps d'incubation de 4, 8, 16, 24, 48 et 72 heures : les plaques obtenues sont représentées sur les figures 2A (pour CE- α) et 2B (pour CE-β). Les dépôts, comme pour les figures 1 et 3A, 3B ont été faits au même niveau repéré par un trait mixte en bas des pistes. Pour un délai d'incubation court, on voit que le composé CE n'a pas le temps d'être beaucoup transformé en composé CT ; mais si le temps d'incubation augmente, la ChEH transforme de plus en plus le composé CE en composé CT, de sorte que les taches CT sont plus denses alors que les taches CE deviennent plus claires ; et simultanément, lorsque le composé CT apparaît, on voit apparaître les « troisièmes taches » correspondant à un métabolite M.

La demanderesse a donc considéré comme vraisemblable que le métabolite M de la « troisième tache » était un dérivé du composé CT.

### Essai 4

Pour vérifier les conclusions tirées des exemples 1 à 3, on a incubé des cellules MCF7 avec le composé CT [¹⁴C] pendant des temps de 24, 48 et 15 72 heures en utilisant les procédures ci-dessus définies. Les extraits cellulaires ont ensuite été obtenus comme indiqué à l'essai 1 et déposés sur une plaque de chromatographie (voir figure 4). La piste de gauche de la plaque reçoit un dépôt de CE[¹⁴C] et la piste voisine reçoit un dépôt de CT[¹⁴C], comme témoins de migration ; les trois autres pistes correspondent aux extraits cellulaires testés après incubation. On constate que, pour une incubation d'une durée de 24 heures, on voit sur la piste de l'extrait cellulaire une tache correspondant à celle du métabolite voisin de la tache du composé CE [¹⁴C]. Cette tache est d'autant plus dense que le temps d'incubation est plus grand et plus la tache du métabolite M est sombre, plus celle du composé CT s'éclaircit.

Ceci confirme que le métabolite est bien un produit de transformation du composé CT.

### Essai 5

Pour identifier la structure chimique du métabolite apparu dans les essais 1 à 4, on a utilisé une technique en plusieurs étapes. Des cellules MCF7 ont été ensemencées à 0,4 10⁶ cellules par boîte de Pétri (diamètre 100 mm) dans 10 ml du milieu défini à l'essai 1. Trente six heures après l'ensemencement, les cellules ont été incubées, à une concentration de 10 µM, pour les unes dans du CEα ou pour les autres dans du CEα[¹⁴C] obtenu comme indiqué précédemment. Après soixante douze heures, les cellules sont lavées par du PBS (tampon phosphate) froid, puis sont grattées dans du PBS froid et centrifugées à 1000 tours/mn pendant 5 minutes à 4°C. Le culot cellulaire et le milieu sont extraits par la méthode de Folch modifiée (voir la référence déjà fournie en page 9 du présent texte).

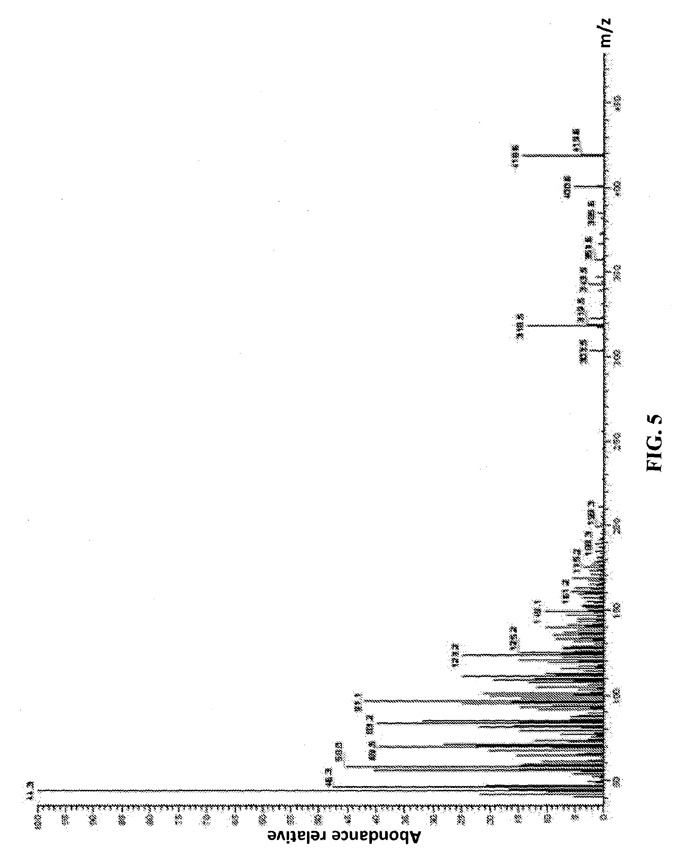

La phase organique est évaporée, resuspendue dans du méthanol puis passée sur une cartouche RP C18 (Sep-Pack de la société Waters). La cartouche est ensuite lavée au méthanol. Après évaporation, le résidu est dissout dans 20µl d'éthanol puis purifié par HPLC en phase inverse (colonne hydrophobe « Ultrasep ») en utilisant des conditions isocratiques : CH₃OH/H₂O (95/5 en volume) à 0,7 ml/mn. Des fractions de 1 mn sont collectées à la sortie de la colonne et la radioactivité est décomptée afin de déterminer les temps de rétention des composés marqués et notamment du métabolite issu du CEα[¹⁴C]. Les fractions radioactives sont analysées par chromatographie sur couche mince en utilisant l'acétate d'éthyle comme solvant de migration. Les fractions HPLC d'intérêt issues des cellules MCF7 traitées par le CEα froid sont analysées par spectrographie de masse en impact électronique (70 ev) et ionisation chimique (voir le spectre sur la figure 5).

On a ainsi déterminé que le CT produit par les cellules MCF7 a une masse de 420 et un comportement chromatographique similaire à celui du CT commercial. La spectrométrie de masse du métabolite existant dans les extraits de cellules MCF7 et purifié comme indiqué ci-dessus, donne une masse de 418 soit une perte de 2 unités de masse par rapport au composé CT. Comme on l'a vu à partir des essais 1 à 4, le métabolite provient de la biotransformation du composé CT : la différence de masse correspond donc à une perte de 2 atomes d'hydrogène. Ceci suggère l'apparition d'une fonction cétone ou l'apparition d'une double liaison sur le composé CT. L'analyse en infrarouge montre l'apparition d'une bande caractéristique d'une fonction cétone, ce qui démontre la formation du 6-oxo-cholestan-3ß,5α-diol (OCDO). La structure du composé OCDO correspond à un produit de déshydrogénation du composé CT sur le groupement hydroxyl porté sur le carbone 6 du noyau. Les propriétés chromatographiques et le profil de fragmentation en spectrométrie de masse du métabolite sont identiques à ceux du standard commercial fourni par la société « Steraloïds » pour le composé OCDO, ce qui démontre l'identité entre ces deux molécules.

### Méthodes de diagnostic du cancer

L'invention concerne donc une méthode pour détecter l'état oncogénique de cellules prélevées à partir d'un échantillon obtenu chez un sujet, caractérisée en ce qu'elle comprend une étape où l'on détermine la quantité du composé OCDO de formule 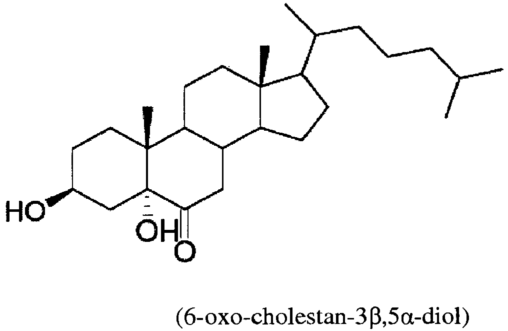
HO
OH
O
(6-oxo-cholestan-3β,5α-diol)
 présente dans les cellules prélevées.

Il est aussi décrit une méthode de diagnostic pour détecter l'état oncogénique de cellules prélevées à partir d'un matériau biologique provenant d'un sujet humain ou d'un animal mammifère, caractérisée en ce que l'on détermine par un moyen révélateur si les cellules prélevées contiennent, en quantité significative, le composé OCDO, et que, si cette détermination est positive, on en déduit que lesdites cellules sont dans un état oncogénique. L'invention concerne également une méthode pour diagnostiquer un cancer chez un sujet, comprenant une étape de détection de l'état oncogénique de cellules d'un échantillon obtenu chez un sujet selon la méthode de détection de l'invention dans laquelle une quantité d'OCDO supérieure à une valeur de référence mesurée dans les cellules d'un échantillon de sujet sain est indicative d'un état oncogénique des cellules dudit échantillon.

Selon l'invention, la quantité d'OCDO dans les cellules de l'échantillon du sujet testé est comparée à une valeur de référence, ladite valeur de référence étant mesurée dans les cellules d'un échantillon d'un sujet sain dans les mêmes conditions expérimentales que pour la mesure de la quantité d'OCDO des cellules de l'échantillon du sujet testé. Une quantité d'OCDO significativement améliorée par rapport à la valeur de référence est alors indicative d'un état oncogénique des cellules dudit échantillon. Par « significativement améliorée » on entend une valeur statistiquement supérieure à la valeur de référence (p<0.05).

Les méthodes selon l'invention permettent typiquement de pronostiquer l'évolution d'une tumeur chez un sujet, et cela a un stade précoce de l'évolution de la maladie. Si les cellules de l'échantillon du sujet présentent une quantité d'OCDO inférieure ou égale à une valeur de référence, cela est indicatif d'un bon pronostic et d'une tumeur bénigne. Au contraire, si les cellules de l'échantillon du sujet présentent une quantité d'OCDO supérieure à une valeur de référence, cela est indicatif d'un mauvais pronostic et d'une tumeur maligne.

Typiquement, la quantité d'OCDO est déterminée à l'aide d'un moyen révélateur.

Dans un mode de réalisation de l'invention, la quantité du composé OCDO est déterminée (mesurée) dans un extrait liquide desdites cellules. Typiquement, cet extrait liquide est obtenu en lysant les cellules puis en séparant les fractions solide et liquide, par exemple par centrifugation. La fraction liquide constitue ledit « extrait liquide » de cellules.

On peut repérer la présence d'OCDO dans l'extrait liquide par chromatographie sur couche mince, la présence d'OCDO étant alors détectée sur la plaque de chromatographie par un moyen révélateur approprié. On peut prévoir que le moyen révélateur consiste en une modification chimique de l'OCDO lui permettant de s'immobiliser sur une protéine de transport, que l'on détecte ultérieurement par des anticorps monoclonaux. Selon une variante, le moyen révélateur est un marquage radioactif des cellules prélevées effectué avant la chromatographie, la révélation se faisant sur la plaque par quantification de la radioactivité.

On a constaté que les cellules ont un état oncogénique, dès lors qu'un extrait liquide desdites cellules présente une concentration d'OCDO supérieure à 1 µM. On peut doser la concentration d'OCDO dans l'extrait par chromatographie en phase liquide à haute pression (HPLC). On peut aussi doser cette concentration par une chromatographie en phase gazeuse suivie d'une spectrographie de masse.

Dans un mode de réalisation particulier, la quantité d'OCDO dans l'extrait liquide est déterminée par chromatographie sur couche mince, le composé OCDO étant détecté sur la plaque de chromatographie par un moyen révélateur approprié.

Il est aussi décrit une utilisation du composé OCDO comme marqueur de l'état oncogénique d'un échantillon obtenu chez un sujet.

L'invention concerne également une utilisation du composé OCDO comme marqueur de l'état oncogénique d'un matériau biologique provenant d'un sujet humain ou d'un animal mammifère.

L'invention concerne également une utilisation du composé OCDO présent dans des cellules d'un échantillon obtenu chez un sujet comme marqueur de l'état oncogénique desdites cellules.

Il est aussi décrit le composé OCDO pour une utilisation dans une méthode de diagnostic du cancer pratiquée sur le corps humain ou animal.

### Méthodes de suivi du traitement d'un sujet atteint d'un cancer

Il est également décrit une méthode pour suivre la réponse à un traitement d'un sujet souffrant d'un cancer, ladite méthode comprenant les étapes de déterminer le diagnostic du sujet avant et au cours du traitement en utilisant la méthode de diagnostic selon l'invention, un changement de diagnostic du sujet au cours du traitement étant indicatif d'une réponse du sujet audit traitement.

L'invention concerne aussi une méthode pour suivre la réponse à un traitement d'un sujet souffrant d'un cancer, ladite méthode comprenant les étapes de détection, avant et au cours du traitement, de l'état oncogénique de cellules d'un échantillon obtenu chez ledit sujet selon la méthode de détection de l'invention; un changement de l'état oncogénique du sujet au cours du traitement étant indicatif d'une réponse du sujet audit traitement.

Selon l'invention, les prélèvements des échantillons avant et au cours du traitement sont réalisés dans les mêmes conditions expérimentales.

### Méthodes de screening de médicaments anticancéreux

L'invention a également pour objet une méthodepour évaluer l'efficacité d'un médicament pour traiter un cancer chez un sujet atteint par ledit cancer, comprenant:
(a) une étape de détection de l'état oncogénique de cellules d'un échantillon obtenu chez un sujet selon la méthode de détection de l'invention où on dose la concentration D1 d'OCDO dans un extrait liquide des cellules d'un échantillon obtenu chez ledit sujet;
(b) après un temps de traitement thérapeutique, une étape de détection de l'état oncogénique de cellules d'un échantillon obtenu chez un sujet selon la méthode de détection de l'invention où on dose la concentration D2 d'OCDO dans un extrait liquide des cellules d'un échantillon obtenu chez ledit sujet de la même manière qu'à l'étape (a);
(c) une étape de comparaison de D1 et D2 ; et
(d) si D2 < D1, on en déduit que le médicament est efficace pour traiter ledit cancer.

Il est également décrit une méthode pour évaluer l'efficacité d'un traitement du cancer chez un sujet atteint par ledit cancer, ladite méthode comprenant les étapes de déterminer le diagnostic du sujet avant et au cours du traitement en utilisant la méthode de diagnostic selon l'invention, un changement de diagnostic du sujet au cours du traitement étant indicatif de l'efficacité dudit traitement pour traiter le cancer.

L'invention concerne aussi une méthode pour évaluer l'efficacité d'un traitement du cancer chez un sujet atteint par ledit cancer, ladite méthode comprenant les étapes de détection, avant et au cours du traitement, de l'état oncogénique de cellules d'un échantillon obtenu chez ledit sujet selon la méthode de détection de l'invention; un changement de l'état oncogénique ou du diagnostic du sujet au cours du traitement étant indicatif de l'efficacité dudit traitement pour traiter le cancer.

Selon l'invention, les prélèvements des échantillons avant et au cours du traitement sont réalisés dans les mêmes conditions expérimentales.

### Méthodes de traitement du cancer

L'invention a également pour objet un inhibiteur de l'OCDO pour une utilisation dans une méthode de traitement d'un cancer chez l'homme ou l'animal, caractérisé en ce qu'il est choisi parmi les inhibiteurs de l'activité cholestérol-époxy hydrolase (ChEH) choisis dans le groupe consistant en le Ro 48-8071 et l'ibogaïne.

Il est aussi décrit une méthode de traitement d'un sujet atteint d'un cancer, ladite méthode comprenant l'administration audit sujet d'une quantité thérapeutiquement efficace d'un inhibiteur d'OCDO.

Selon un mode de réalisation, l'OCDO est dissout dans l'éthanol, la solution obtenue est alors diluée (typiquement au 1/1000) dans un tampon, notamment un tampon phosphate, et la solution diluée ainsi obtenue étant injectée au sujet, typiquement en intra tumoral. Selon l'inhibiteur utilisé, on peut injecter des doses journalières comprises entre 1 µg/kg et 500 µg/kg pendant un temps compris entre 1 et 10 semaines. Toutefois, l'homme du métier est apte à adapter la forme sous laquelle est administré l'inhibiteur d'OCDO, tout comme la posologie et la durée du traitement, selon le patient et la maladie traitée.

Il est décrit que l'inhibiteur d'OCDO peut typiquement être choisi parmi les produits suivants :
- les inhibiteurs d'enzyme(s) impliqués dans la biosynthèse du cholestérol tels que la lovastatine, inhibiteur Ro 48-8071, le U18666A, le AY-9944, le triparanol, la terbinafine, le SKF-525A;
- les inhibiteurs des cytochromes P 450, les lipoxygènases et les antioxydants actifs sur l'époxydation du cholestérol tels que le ketoconazole et la vitamine E ;
- les inhibiteurs de l'activité cholestérol-époxy hydrolase (ChEH) tels que le PBPE, le PCPE, le tesmilifène, la dendrogenine A (DDA), le tamoxifène, le 4 hydroxytamoxifène le raloxifène, le nitromiphène, le clomiphène, le RU 39411, le BD-1008, l'haloperidol, le SR 31747A, l'ibogaïne, le AC-915, le Rimcazole, l'amiodarone, la trifluorperazine, le U18666A, le AY-9944, le triparanol, la terbinafine, le SKF-525A ;
- les inhibiteurs choisis dans le groupe formé par :
   - les antagonistes des récepteurs des oestrogènes ;
   - les ligands des sites de liaison membranaires des anti-oestrogènes (AEBS) ;
   - les ligands des récepteurs σ 1 et 2 et certains amino-alkyl stérols
   - les inhibiteurs du transport intra-cellulaire du cholestérol ; et
   - les inhibiteurs d'enzyme choisis dans le groupe formé par la progestérone et les antagonistes des récepteurs Ahr.

D'autres aspects et avantages de la présente invention sont décrits dans les figures et les exemples suivants, qui doivent être considérés à titre illustratif et comme ne limitant pas la portée de l'invention.

### Brève description des figures

**Fig. 1** **:** Mesure de l'activité ChEH dans les cellules MCF7 et dans des hépatocytes des souris. Les cellules sont incubées en présence de CE[¹⁴C] puis les lipides sont extraits et les stérols sont analysés et séparés en chromatographie sur couche mince sur plaque de silice. La plaque de chromatographie est révélée par autoradiographie. CE : epoxy-5,6-cholestanol, CT : cholestan-3β,5α,6β-triol, M : métabolite M.
**Fig. 2****:** Cinétique de l'activité ChEH dans les cellules MCF7. Les cellules MCF7 sont incubés en présence de CE α[¹⁴C] (A) ou de CE β[¹⁴C] (B) pendant des temps croissants de 4 à 72 heures. Les lipides sont extraits des cellules et les stérols sont analysés et séparés en chromatographie sur couche mince sur plaque de silice. La plaque de chromatographie est révélée par autoradiographie. CE : epoxy-5,6-cholestanol, CT : cholestan-3β,5α,6β-triol, M : métabolite M.
**Fig.3** **:** Inhibition dose-dépendante de l'activité ChEH (3 jours) dans les cellules MCF7 par le Tamoxifène et par le PBPE. Les cellules MCF7 sont incubés en présence de CE α[¹⁴C] (A) ou de CE β[¹⁴C] (B) pendant trois jours en présence de concentrations croissantes de Tamoxifène (A) ou de PBPE (B). Les lipides sont extraits des cellules et les stérols sont analysés et séparés en chromatographie sur couche mince sur plaque de silice. La plaque de chromatographie est révélée par autoradiographie. CE : epoxy-5,6-cholestanol, CT : cholestan-3β,5α,6β-triol, M : métabolite M.
**Fig.4** **:** Transformation du CT en métabolite M. Les cellules MCF7 sont incubés en présence de CT[¹⁴C] (A) pendant 24, 48 et 72 heures. Les lipides sont extraits des cellules et les stérols sont analysés et séparés en chromatographie sur couche mince sur plaque de silice La plaque de chromatographie est révélée par autoradiographie. CE : epoxy-5,6-cholestanol, CT : cholestan-3β,5α,6β-triol. M : métabolite M.
**Fig.5** **:** Spectre de masse du métabolite C. Les cellules MCF7 sont incubées en présence de CE puis les lipides sont extraits puis purifiés par HPLC et la fraction correspondant à l'OCDO est analysée par spectrométrie de masse.

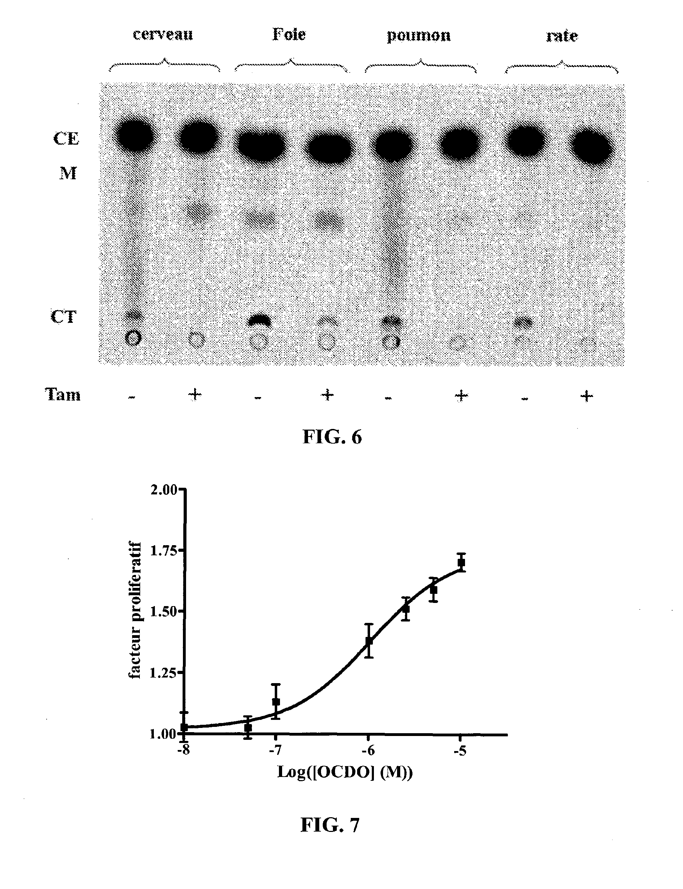

**Fig.6** **:** Analyse de la production d'OCDO dans les tissus sains. Les cellules provenant de différents organes des souris sont incubés en présence de CT[¹⁴C] (A) pendant 48 heures en présence ou en absence de tamoxifène. Les lipides sont extraits des cellules et les stérols sont analysés et séparés en chromatographie sur couche mince sur plaque de silice La plaque de chromatographie est révélée par autoradiographie. CT : cholestan-3β,5α,6β-triol.
M : métabolite M.
**Fig.7** **:** Effet de l'OCDO sur la prolifération cellulaire *in vitro.* Les cellules d'un carcinome médulaire de la thyroïde humain (cellules TT) sont cultivées en présence de concentrations croissantes d'OCDO allant de 0,1 à 5 µM durant 4 jours. Après ce temps d'incubation la quantité de cellule est mesurée et le facteur prolifératif déterminé.

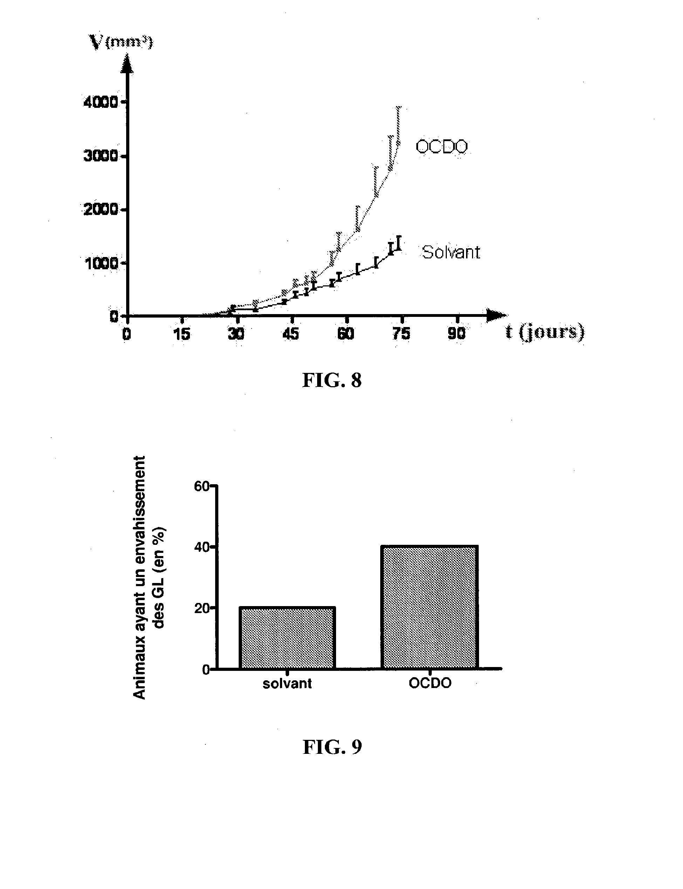

**Fig.8** **:** Effet de l'OCDO sur la progression tumorale *in vivo.* Les cellules TT sont implantée sur des souris nudes. Les souris sont traitées soit avec le solvant véhicule (Solvant) soit par 50µg/kg d'OCDO par injection 1 fois par jour 5 jours sur 7 pendant plusieurs semaines.
Le volume de la tumeur est mesuré et reporté sur le graphe en fonction du temps.
**Fig.9** **:** Analyse des ganglions lymphatiques. La présence de cellule tumorale est recherchée dans les ganglions lymphatiques et comparée entre animaux traités par le solvant véhicule (Solvant) ou l'OCDO.

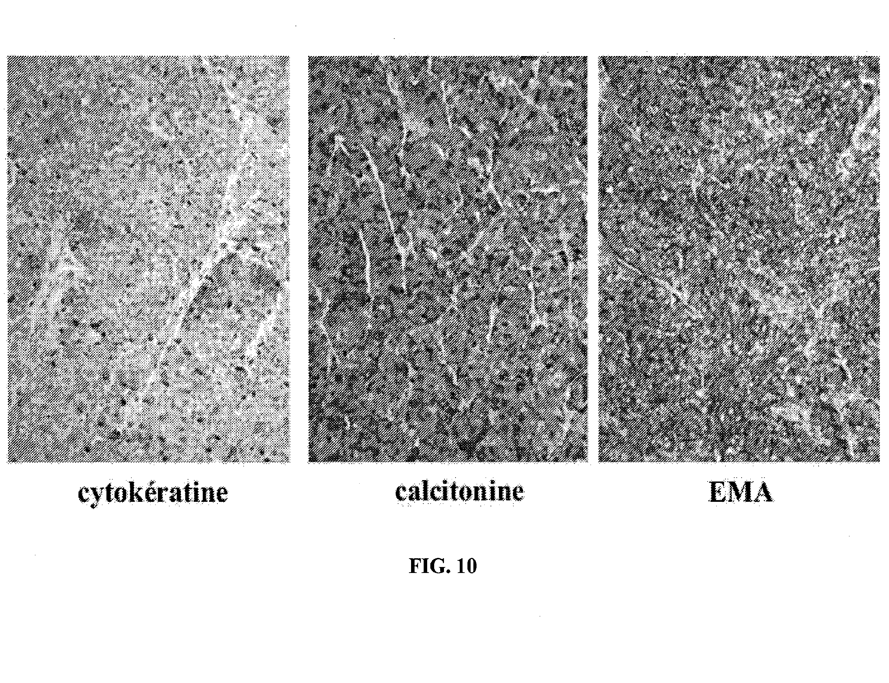

**Fig.10** **:** Analyse histomorphologiques des tumeurs. Les tumeurs des animaux traités par le solvant véhicule (Solvant) ou l'OCDO sont fixées et incubées en présence d'anticorps dirigés contre une cytokeratine, la calcitonine et un antigène de membrane épithéliale (EMA). La révélation est effectuées à l'aide d'un complexe streptavine-biotine-peroxydase suivi d'une incubation avec une solution de diaminobenzidine puis une coloration à l'hématoxiline est réalisée. La présence de coloration brune est révélatrice de l'expression des protéines marqués par les anticorps.

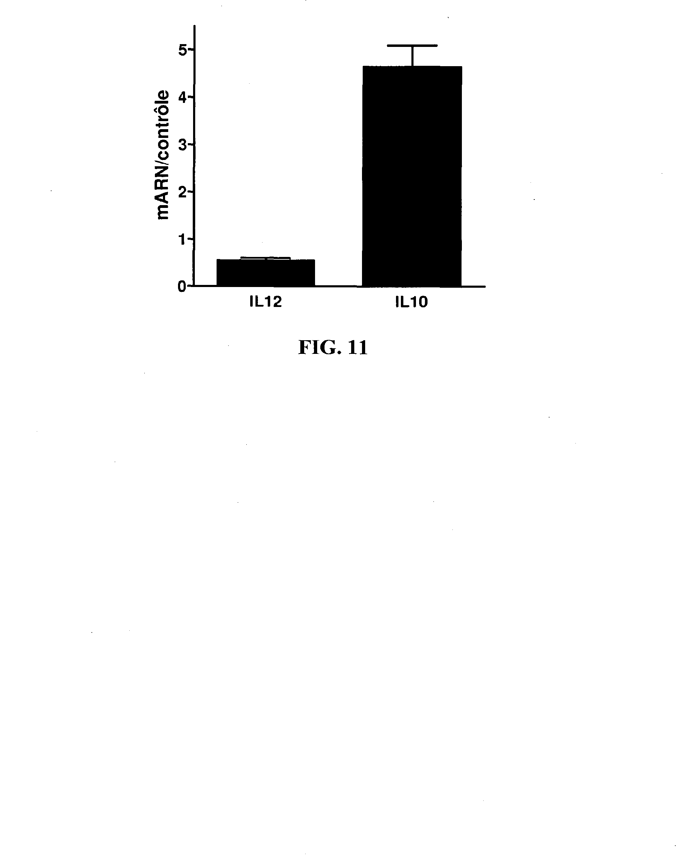

**Fig.11** **:** Effet de l'OCDO sur l'expression de cytokines. Des cellules THP1 sont traitées *in vitro* par 10 µM d'OCDO pendant 4 heures. Les ARNS totaux des cellules sont extraits et l'expression des gènes codant pour l'IL12 et l'IL10 est réalisée et reporté sur la figure.

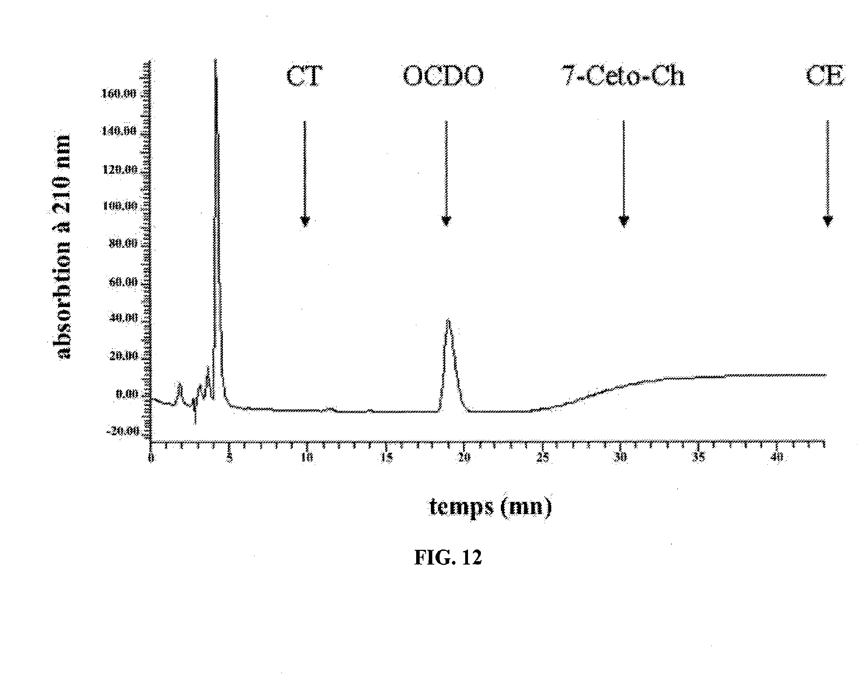

**Fig.12** **:** Analyse d'OCDO par chomatographie liquide à haute pression (HPLC). L'OCDO est séparé des d'autres oxysterols tels que l' epoxy-5,6-cholestanol (CE), le cholestan-3β,5α,6β-triol (CT) et le 7-cétocholesterol (7-ceto-Ch). On utilise une détection UV à 210 nm.

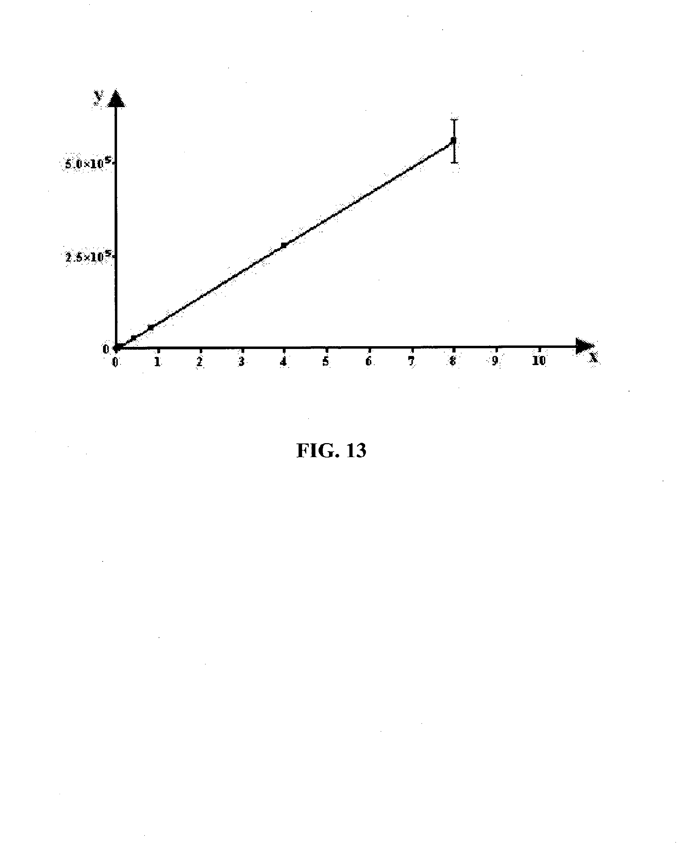

**Fig.13** **:** Courbe de calibration de l'OCDO par HPLC. Des quantités croissantes de 0,1 à 5 µg d'OCDO sont injectées et analysées par HPLC. L'aire des pics correspondant à l'OCDO est reportée en fonction de la masse de produit injecté. Une droite de corrélation est établie à partir du graphe.

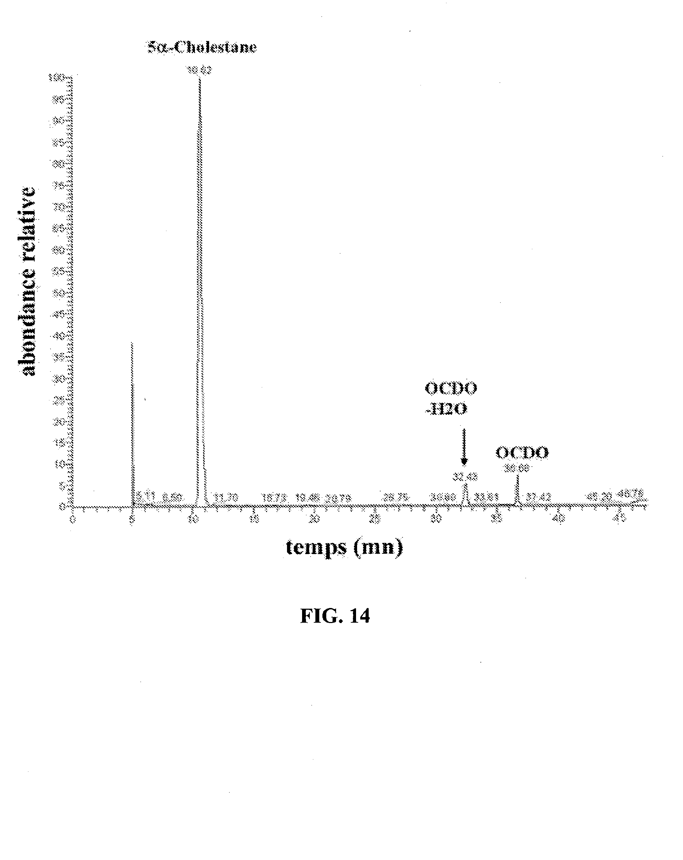

**Fig.14** **:** Analyse de L'OCDO par chromatographie gazeuse (GC). Un mélange de 5α-cholestane et d'OCDO est trimethylsillilé puis analysé par Chromatographie gazeuse couplée à la spectrométrie de masse (GC/MS). On obtient 3 pics. Le premier (10,62 minutes) correspond au 5α-cholestane, le second (32,43 minutes) correspond à l'OCDO ayant perdu une molécule d'H₂O (OCDO - H₂O), le troisième (36,68 minutes) correspond à l'OCDO.

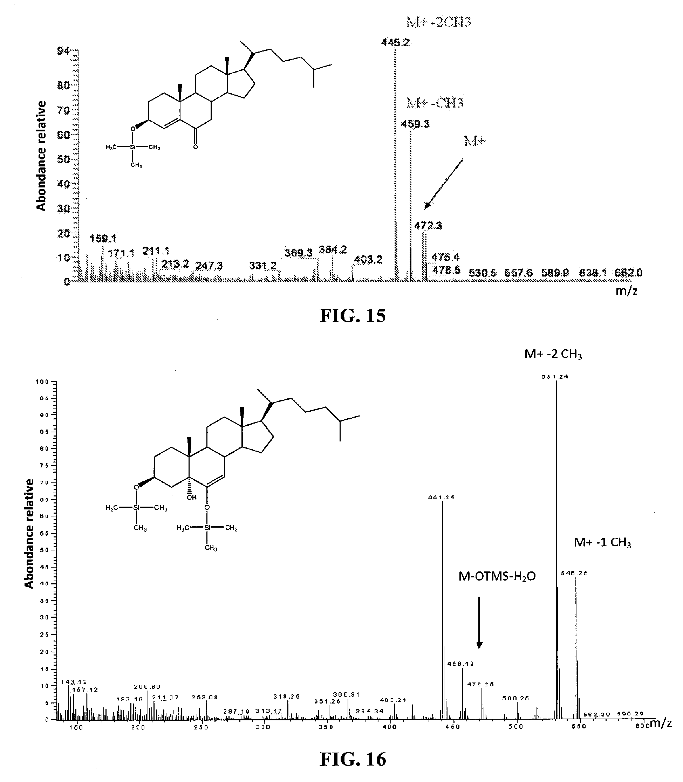

**Fig.15** **:** Analyses en masse du pic sortant à 32,43 minutes en GC. Le profile de fragmentation en masse fait apparaitre un produit de déshydratation de l'OCDO (M+(472,3). La structure du produit obtenu est représentée.
**Fig.16** **:** Analyses en masse du pic sortant à 36,68 minutes en GC. Le profile de fragmentation en masse fait apparaitre un produit de déshydratation de l'OCDO (M+(-CH₃)546). La structure du produit obtenu est représentée.

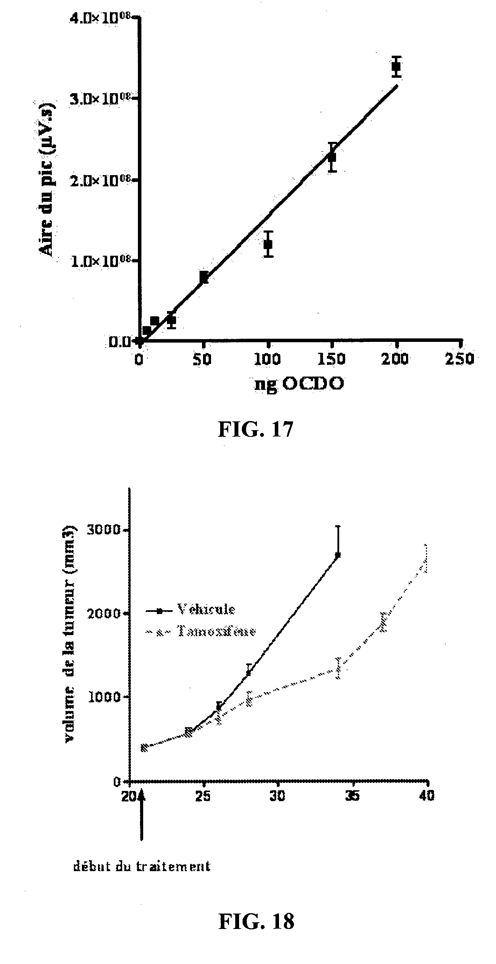

**Fig.17** **:** Courbe de calibration de l'OCDO par GC/MS. Des quantités croissantes de 6,25 ng à 100 ng d'OCDO sont injectées et analysées par GC/MS. L'aire des pics correspondant à l'OCDO est reportée en fonction de la masse de produit injecté. Une droite de corrélation est établie à partir du graphe.
**Fig.18** **:** Inhibition de la pousse tumorale par le Tamoxifène *in vivo.* Des cellules mammaires de type TS/A sont implantées sur des souris BALB/c. Les souris sont traitées par le Tamoxifène (Tamoxifène) ou par le solvant véhicule (véhicule). Le volume des tumeurs est mesurée au cours du temps.

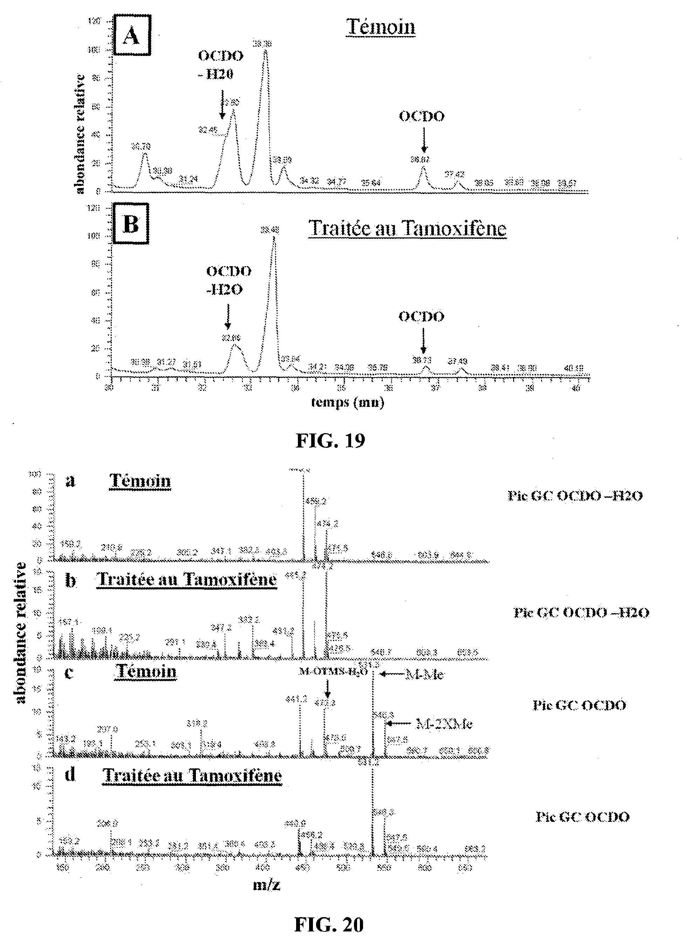

**Fig.19****:** Analyse en GC du contenu en OCDO des tumeurs. Les tumeurs des animaux traités ou non au tamoxifène sont extraites. Les extraits sont analysés par GC/MS. Le profile GC des extraits des aninaux traités par le solvant véhicule (A) révèle la présence de pics correspondant à l'OCDO et à son produit de deshydratation (OCDO-H₂O).
**Fig.20** **:** Analyse en MS des pics GC correspondant à OCDO et OCDO-H₂O. L'analyse en masse confirme la structure des composés présents dans les pics obtenus en GC.

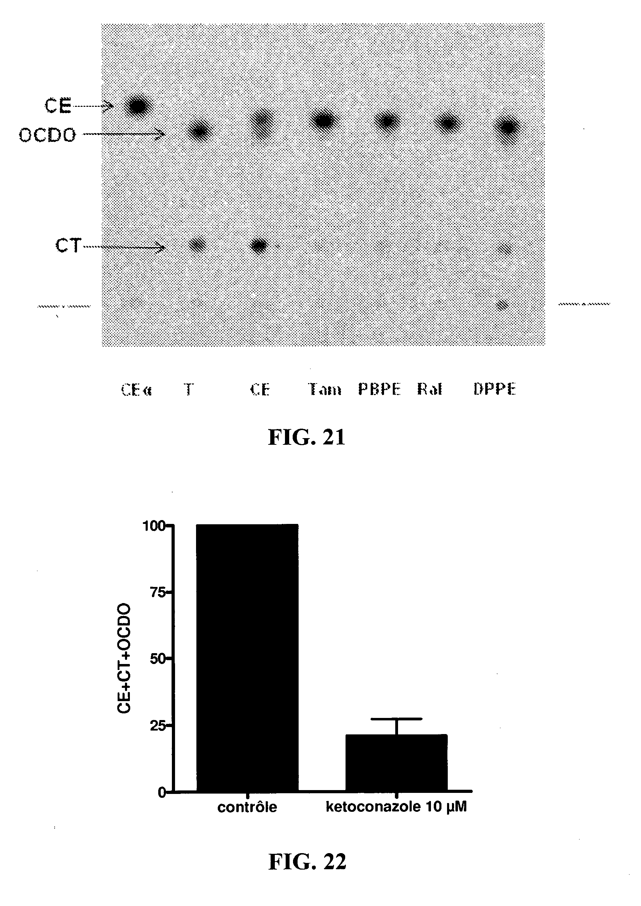

**Fig.21** **:** inhibition de la production d'OCDO dans les cellules MCF7 par des inhibiteurs de ChEH. Les cellules MCF7 sont incubés en présence de CE α[¹⁴C] pendant trois jours en présence du solvant véhicule (T), de cholesterol epoxyde (CE), de Tamoxifène (Tam), de PBPE (PBPE), de raloxiphene (Ral), de tesmilifene (DPPE). La piste notée CE α correspond a un depot de CE α[¹⁴C] utilisé comme standard de migration. Les lipides sont extraits des cellules et les stérols sont analysés et séparés en chromatographie sur couche mince sur plaque de silice. La plaque de chromatographie est révélée par autoradiographie. CE : epoxy-5,6-cholestanol, CT : cholestan-3β,5α,6β-triol.
**Fig.22** **:** inhibition de la production d'OCDO dans les cellules MCF7 par un le Ketoconazole, un inhibiteur général de cytochrome P450. Les cellules MCF7 sont incubés en présence de CE α[¹⁴C] pendant trois jours en présence du solvant véhicule (contrôle) ou de 10 µM de ketoconazole. Les lipides sont extraits des cellules et les stérols sont analysés et séparés en chromatographie sur couche mince sur plaque de silice. La plaque de chromatographie est révélée par autoradiographie et la quantité de CE et d'OCDO quantifiée.

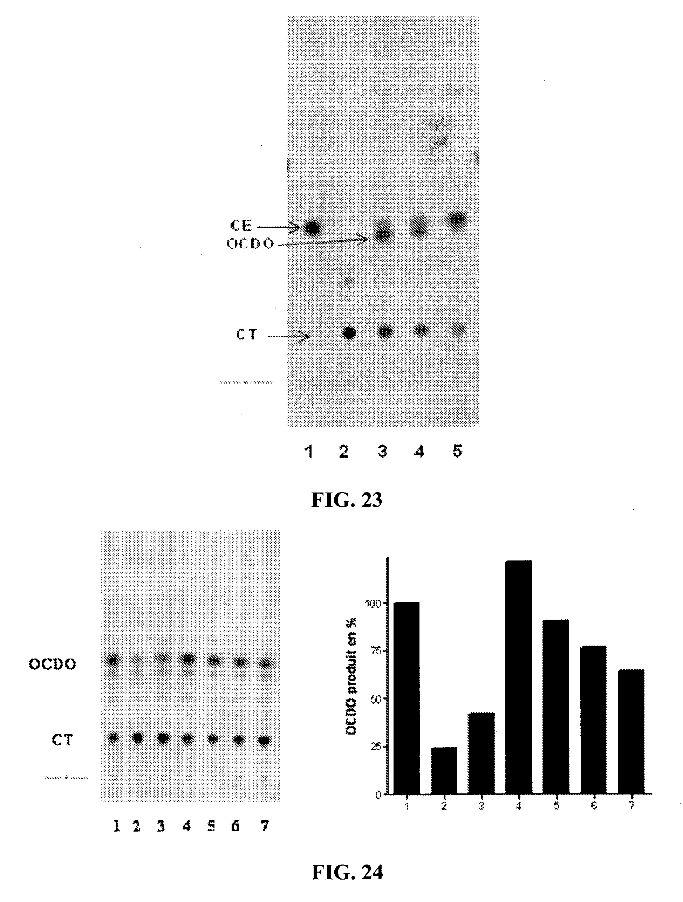

**Fig.23****:** Inhibition de la production d'OCDO dans les cellules MCF7 par un aminoalkylsterol (DDA). La piste 1 correspond a un dépot de CE α[¹⁴C] utilisé comme standard de migration. La piste 2 correspond a un dépot de CT α[¹⁴C] utilisé comme standard de migration. Les cellules MCF7 sont incubés en présence de CE α[¹⁴C] pendant 48 heures en présence du solvant véhicule (piste 3), de 0.1 µM de DDA (piste 4), de 1µM de DDA. Les lipides sont extraits des cellules et les stérols sont analysés et séparés en chromatographie sur couche mince sur plaque de silice. La plaque de chromatographie est révélée par autoradiographie. CE : epoxy-5,6-cholestanol, CT : cholestan-3β,5α,6β-triol.
**Fig.24** **:** inhibition de la production d'OCDO dans les cellules MCF7 par des modulateurs de transport intracellulaire du cholesterol et des modulateurs de récepteurs AhR: Les cellules MCF7 sont incubés en présence de CT α[¹⁴C] pendant 24 heures en présence du solvant véhicule (piste 1), de progesterone (piste 2), de U18666A (piste 3), de TCDD (piste 4), de benzo(A)pyrene (piste 5), de resveratrol (piste 6) de PDM2 (piste 7). Les lipides sont extraits des cellules et les stérols sont analysés et séparés en chromatographie sur couche mince sur plaque de silice. La plaque de chromatographie est révélée par autoradiographie. CT : cholestan-3β,5α,6β-triol.

### Exemple 1 : L'OCDO est un marqueur de l'état tumoral d'une cellule.

On a testé, par la méthode décrite à l'exemple 4 ci-après, la présence d'OCDO sur de nombreuses lignées cellulaires tumorales afin de d'établir que ce marqueur est observable dans différents types cellulaires chez l'homme, le rat ou la souris. La production de l'OCDO a été détectée dans chacune des lignées cellulaires tumorales testées comme indiqué dans le tableau suivant :

| **Lignée cellulaire** | **Type** | **Production d'OCDO** |
|---|---|---|
| MCF7 | Carcinome mammaire humain | + |
| MCF7/TamR | Carcinome mammaire humain résistant au tamoxifène | + |
| MDA-MB-231 | Carcinome mammaire humain | + |
| TS/A | Carcinome mammaire murin | + |
| A-549 | Carcinome pulmonaire humain | + |
| B16-F10 | Mélanome murin | + |
| SK-MEL-28 | Mélanome humain | + |
| U-937 | Leucémie myéloïde humain | + |
| J 774 | Leucémie myéloïde murine | + |
| THP.1 | Leucémie monocytaire aigüe humaine | + |
| HT-29 | Carcinome du colon humain | + |
| HeLa | Carcinome utérin humain | + |
| C6 | Gliome de rat | + |
| SK-N-SH | Neuroblastome humain | + |
| Saos-2 | Ostéosarcome humain | + |
| P19 | Carcinome embryonnaire murin | + |
| TT | Carcinome médulaire de la thyroïde humain | + |

Les demanderesses, ayant constaté que l'OCDO se retrouve dans toutes les cellules tumorales testées, ont conclu que l'OCDO constituait un marqueur de l'état tumoral des cellules.

Cependant, à titre préalable, et de façon analogue à ce qui a été fait dans les essais 1 à 5 précédents, on a évalué la transformation du composé CE dans des cellules de différents tissus sains de souris dans des conditions qui permettaient de détecter l'OCDO (voir le résultat sur la figure 6). On a incubé lesdites cellules avec le composé CEα[¹⁴C] et on a observé en chromatographie sur couche mince de silice les taches radioactives qui apparaissent avec (+) ou sans (-) incubation au tamoxifène. On constate que si le tamoxifène est présent, le composé CT n'apparaît plus, ce qui correspond bien à l'inhibition de la ChEH ; en l'absence d'incubation au tamoxifène, le composé CT apparaît mais on ne note aucune présence, au voisinage du composé CE, de tache correspondant à la présence d'OCDO.

Il est donc claire que l'OCDO n'est pas produit dans un tissu normal ; sa détection constitue donc bien un élément prédictif de l'état tumoral des cellules ; l'OCDO est un marqueur de l'état oncogénique d'une cellule.

### Exemple 2 : l'OCDO est carcinogène

A partir des essais précédemment décrits et de l'exemple 1, les demanderesses ont pensé, selon l'invention, que la présence de l'OCDO dans les cellules en situation tumorale, pouvait être liée au fait que cette molécule pourrait avoir, par elle-même, un potentiel carcinogène.

Un tel potentiel n'a jamais été rapporté antérieurement dans la littérature.

Le cholestérol n'a pas de propriétés mutagènes. Les précurseurs de l'OCDO et les épimères de Cholestérol époxydes ont été cités comme de faibles mutagènes dans des cellules de mammifères (Peterson AR, Peterson H, Spears CP, Trosko JE and Sevanian , Mutation Research, vol 203 p. 355-366, 1988). Les sterol-epoxides n'ont pas montré de propriétés mutagènes dans le test d'Ames réalisé sur des bactéries (Smith LL et al., Mutation Research, vol 68 p. 23-30, 5 1979 ; Ansari GAS et al., Vol 20, p. 35-41, 1982). Ces observations ont été confirmées récemment (Cheng YW et al., Food and Chemical Toxicology, vol 43, p. 617-622, 2005). Certes, on a trouvé des homologies structurales de l'OCDO avec des promoteurs de tumeur tel que le TPA (12-tetradecanoylphorbol 13-acetate) (Endo Y. et al., Chem. Pharm. Bull. (Tokyo), vol 42 (3) p. 462-469, 1994). Mais personne n'a décrit ni même suggéré que l'OCDO pourrait avoir des propriétés d'activation des PKC, ni de promotion de tumeurs chez l'animal de laboratoire ; on a uniquement montré que cette molécule se fixait sur une protéine de fixation des esters de phorbol (Endo Y., Biochem. Biophys. Res. Comm., vol 194, p. 1529-1535, 1993).

Sur le plan cellulaire, on a seulement indiqué que l'OCDO paraît :
- 1) inhiber la formation de rosette lymphocyte T provenant de sérum humain (Streuli R.A. et al., J. Immunol., Vol. 123 (6)n, p.2897-2902) ;
- 2) inhiber la mobilité des leucocytes (Gordon L, Proc. Natl. Acad. Sci. USA, vol 77(7), p.4313-4316, 1980 ;
- 3) induire la toxicité des cellules NK chez la souris (Kucuk O. et al., Cell. Immunol., 139, p. 541-549, 1992) ;
- 4) inhiber l'activité cytolytique produit par les lymphocytes T chez la souris (Kucuk O et al., Lipids, Vol 29(9), p. 657-660, 1994), ces effets étant observés à des concentrations comprises entre 1 et 20 µM.

Par le présent exemple 2, les demanderesses ont établi, dans le cadre la présente invention, que l'OCDO a des effets sur le développement tumoral, qui n'étaient aucunement suggérés à l'homme de métier par l'état de la technique.
a) Dans cet exemple 2, on a utilisé la lignée cellulaire humaine TT de carcinome médullaire de la thyroïde (American Tissues and Cells Collection). Cette lignée est cultivée dans un milieu F12K modifié par Kaighn (vendu par la société « Invitrogen »), contenant 10 % en poids de sérum de veau foetal et 2,5 ml d'une solution d'antibiotiques (pénicilline/streptomycine) à 50 UI/g. L'OCDO testé provient de la société « Steraloids ». Pour étudier in vitro l'effet de l'OCDO sur la croissance cellulaire, les cellules TT sont ensemencées sur des plaques six puits (200 000 cellules par puit) dans un milieu F12K tel que défini ci-dessus. Les cellules TT sont traitées tous les deux jours soit par un solvant (éthanol à 0,1% dans du tampon phosphate PBS) soit par des quantités d'OCDO dans le solvant conduisant à des concentrations de 0,1 ; 1 ; 2,5 ; ou 5 µM. Les cellules sont comptées 4 jours après l'ensemencement. Comme on le voit sur la figure 7, la prolifération des cellules TT est augmentée de façon concentration-dépendante par le traitement avec l'OCDO, quand on compare, comme référence, à la prolifération des cellules traitées par le solvant. A la concentration de 5 µM en OCDO, un facteur d'induction de prolifération de 1,5 est mesuré après 4 jours de traitement ; l'induction de la prolifération est donc établie in vitro pour des concentrations d'OCDO comprises entre 100 nM et 5 µM. Le facteur de stimulation de la prolifération est comparable à celui des oestrogènes, qui sont des molécules ayant des propriétés mitogènes sur des cellules tumorales mammaires (voir : de Medina et al., J Pharmacol Exp Ther, vol 319, 2006 : p. 139-149*).*
b) Les demanderesses ont également étudié in vivo l'effet de l'OCDO sur le développement tumoral. Pour l'injection à l'animal, les cellules TT précédemment définies dans cet exemple sous a), sont récupérées avec de la trypsine, lavées deux fois et suspendues dans un tampon phosphate PBS. Les cellules TT (environ 4.10⁶ cellules/0,1 ml) sont ensuite injectées en sous-cutané dans le flanc de souris femelles « nude nu/nu Swiss » de 6 semaines (fournies par Charles River). Les animaux sont traités par voie sous-cutanée une fois par jour, 5 jours sur 7, soit avec l'OCDO à la dose de 50 µg/kg (groupe traité) soit avec le solvant (groupe contrôle) sur une période de 110 jours (le solvant utilisé est de l'éthanol à 0,1 % dans du tampon phosphate (PBS)). Les animaux (5 ou 10 souris par groupe) sont suivis régulièrement pour mesurer le développement des tumeurs. Le volume des tumeurs est calculé selon la formule L x 1² x 0,5 où L est la longueur et 1 est la largeur de la tumeur. La figure 8 représente le volume V des tumeurs en fonction du temps de traitement t. Le traitement par l'OCDO accélère significativement la croissance de la tumeur ; le volume de la tumeur chez les animaux traités avec l'OCDO est presque trois fois plus important que pour les animaux témoins traités avec le solvant.

On a euthanasié les animaux après 75 jours de traitement et on a prélevé la tumeur ainsi que les différents organes pour être analysés en histologie. On a observé un envahissement des ganglions lymphatiques (GL) chez les animaux traités par l'OCDO deux fois supérieur à celui des animaux traités par le solvant (voir figure 9).

On a pratiqué une analyse histomorphologique. Pour ce faire, les tumeurs des souris traitées par l'OCDO ou par le solvant ainsi que différents organes (ganglions lymphatiques, poumon et foie) sont prélevés, fixés dans 10 % de formaline neutre tamponnée et inclus dans des blocs de paraffine. Pour ces analyses, les coupes sont marquées avec de l'hématoxyline et de l'éosine. L'immunomarquage est réalisé avec des anticorps dirigés contre différents antigènes humains associés aux carcinomes médullaires de la thyroïde. Les anticorps utilisés sont l'anticorps polyclonal anti-calcitonine (Dako SAS, Trappes, France, 1:1000), l'anticorps monoclonal anti-cytokératine (Dako clone AE1/AE3, 1:50) et l'anticorps monoclonal anti-antigène de membranes épithéliales (EMA) (Dako clone E29, 1:50). L'immunomarquage des coupes de paraffine est précédé par une technique de récupération d'antigènes par chauffage dans un tampon citrate (10 mM, pH 6) soit 15 deux fois pendant 10 minutes dans un four à micro-ondes (750 W) pour l'anticorps anti-CEA, soit dans un bain d'eau à 95°C pendant 40 minutes pour les anticorps anti-calcitonine et anti-EMA.

Après incubation avec les anticorps ci-dessus définis, les coupes sont immunomarquées avec le complexe streptavidine-biotine-peroxidase (StreptABComplex/HRP,Dako) suivi par une solution chromogène de diaminobenzidine (DAB), puis sont colorées avec de l'hématoxyline.

Les contrôles négatifs sont réalisés par incubation dans une solution tampon ne contenant pas l'anticorps primaire. Les résultats obtenus par cette analyse histologique sont représentés sur la figure 10 pour chacun des trois anticorps utilisés. On constate que les tumeurs issues des animaux traités par l'OCDO sont bien des carcinomes médullaires de la thyroïde humains, ce qui confirme l'envahissement des ganglions lymphatiques par des cellules issues de la tumeur.

Les demanderesses ont donc établi que l'OCDO a une activité mitogène in vivo en stimulant la pousse tumorale implantée chez l'animal de laboratoire.

**Exemple 3 :** l'OCDO stimule IL10 et réduit IL12, ce qui explique son action carcinogène. Les demanderesses, ayant établi, selon l'invention, que l'OCDO stimule la capacité d'invasion des cellules cancéreuses in vivo, ont confirmé cet effet de l'OCDO en étudiant in vitro l'expression des cytokines sur des cellules THP1 traitées in vitro par l'OCDO.

Les cellules THP1 (lignée cellulaire myéloïde humaine fournie par l'ATCC) sont cultivées avec un milieu de culture de type DMEM (Dubelco modified eagle medium) complété par 10 % de sérum de veau foetal et un mélange d'antibiotiques. Les cellules THP1 sont traitées par 10 µM d'OCDO pendant 4 heures. Les ARN sont extraits selon une procédure classique. Les ADN complémentaires sont produits, puis utilisés pour mesurer l'expression des gènes d'intérêt. On a étudié l'expression des gènes codant l'interleukine 12(IL12) et l'interleukine 10 (IL10), qui représentent un couple de cytokines aux propriétés antagonistes. L'IL12 est une cytokine immunostimulante alors que l'IL10 est une cytokine immunosuppressive. Le rapport d'expression de ces deux cytokines permet d'évaluer le potentiel immunosuppresseur ou immunostimulant de la cellule et sa capacité à favoriser la progression tumorale ou au contraire à la ralentir.

Les amorces utilisées correspondent aux séquences humaines de l'IL10 et de l'IL12 et sont les suivantes :
IL10 sens : AAA-CCA-AAC-CAC-AAG-ACA-GAC (SEQ ID NO:1),
IL10 Anti Sens : GCT-GAA-GGC-ATC-TCG-GAG (SEQ ID NO:2),
IL12 Sens : CTA-TGG-TGA-GCC-GTG-ATT-GTG (SEQ ID NO:3),
IL12 Anti Sens : TCT-GTG-TCA-TCC-TCC-TGT-GTC (SEQ ID NO :4).

Les résultats sont représentés sur la figure 11: l'OCDO stimule l'expression de la cytokine immunosuppressive IL10 et réduit l'expression de la cytokine IL12. Ce mécanisme permet d'expliquer la stimulation par l'OCDO de la capacité d'invasion des cellules tumorales constatée par l'exemple 2.

### Exemple 4 : Dosage de l'OCDO

### A) Dosage par HPLC

On a séparé et dosé l'OCDO par une méthode de chromatographie liquide à haute pression HPLC(95 MeOH/5 H₂O ; 0,7 ml/mn ; colonne Ultrasep ES 6µM de 250 x 4, C18 (Bishoff, Leonberg, Allemagne)). Le chromatographe est un appareil de la société Perkin Elmer ; il comporte une pompe série 200 et un détecteur à barrettes de diodes de type 200.

L'appareil est équipé d'un ordinateur « PC », qui met en oeuvre le programme Turbochrome ^{(™)} permettant le pilotage des appareils et le traitement des données.

On réalise un extrait cellulaire de cellules tumorales MCF7 cultivées comme indiqué à l'essai 1 : on lyse environ 60 millions de cellules pour obtenir 25 µL d'extrait liquide après une centrifugation de 10 mn à 1200 t/mn. On passe 20 µL de cet extrait dans la colonne du chromatographe.

Le chromatogramme résultant est fourni sur la figure 12 : on voit que l'OCDO est séparé du CT, du CE et du céto-cholestérol (7-Ceto-Ch). Le temps de rétention de l'OCDO est de 19 mn.

On effectue un étalonnage pour relier la mesure de la surface du pic OCDO ainsi obtenu et le poids de l'OCDO contenu dans l'échantillon.

Cet étalonnage est réalisé à l'aide de solutions éthanoliques d'OCDO vendu par la société « Steraloids », de concentrations croissantes. On utilise un volume fixe de 20 µL d'échantillon. Les quantités suivantes d'OCDO ont été injectées : 80 ng, 0,4 µg, 0,8 µg, 4µg et 8 µg. L'aire des pics correspondant à l'OCDO a été mesurée par intégration à l'aide du logiciel Turbochrome ^{(™)} ; ces valeurs (y) ont été reportées sur le graphe de la figure 13 en fonction des masses d'OCDO (x) correspondantes. On obtient une droite de corrélation convenable (y=69430x+1381 ; r²=0.999 ; n=6x3 ; p<0,0001 ; x en µg d'OCDO ; y en µV.s) permettant de quantifier l'OCDO pour des masses comprises entre 40 ng à 80 µg. Cette méthode permet de quantifier l'OCDO pour des quantités comprises entre 0,1 et 5 µg de molécules.

### B) Dosage par chromatographie en phase gazeuse couplée à une spectrographie de masse (GC/MS)

Un mélange de 5α-cholestane et d'OCDO est triméthylsilylé selon la méthode décrite par Kedjouar (voir Kedjouar et al, J Biol Chem, 2004, vol 279 n° 32, p. 34048-61). L'échantillon est traité par 20 un mélange de N,Obis(triméthysilyl)trifluoroacétamide/pyridine (50:50, v/v) pendant 30 mn à une température de 60°C. Les réactifs sont évaporés sous un flux d'azote et les dérivés triméthylsilylés (TMS) sont dissous dans l'hexane. Ces analyses GC/MS sont réalisées sur un appareil de type 4890 « Hewlett Packard system » équipé d'une colonne capillaire de silice RTX-50 (30mx0.32mm diamètre interne, film d'épaisseur 0.1 µm ; Restek). La température du four a été programmée à 230°C pendant 1 minute, ensuite de 230 à 240°C à une vitesse d'augmentation de 1° par minute pendant 10 minutes, de 240°C à 250°C à une vitesse d'augmentation de 3° par minute, ensuite jusqu'à 290°C à une vitesse d'augmentation de 45C/mn puis à 330°C en 1 minute.

L'injecteur et le détecteur étaient à 310°C et 340°C respectivement. Le profile GC obtenu est représenté sur la figure 14. On obtient trois pics. Le premier correspond au 5α-cholestane, qui a un temps de rétention de 10,62 minutes. Le deuxième a un temps de rétention de 32,43 minutes et donne, en analyse par spectrométrie de masse avec fragmentation par impact électronique, un résultat qui correspond à un produit de déshydratation de l'OCDO (M+(472.3), M+ moins un groupement CH₃ et M+ moins 2 groupements de CH3 (voir figure 15). Enfin, le dernier pic (temps de rétention de 36,68 minutes) correspond à l'OCDO et son profil de fragmentation en masse est donné sur la figure 16. Ce profil détermine les masses de 546.2 (M+moins un CH₃), 531.2 (M+moins deux CH₃), 472,2 (M+moins H₂O et moins un groupement OTMS).

L'étalonnage de la méthode est réalisé à l'aide de solutions éthanoliques d'OCDO de concentrations croissantes. Les quantités suivantes d'OCDO ont été injectées 6.25 ng, 12.5 ng, 50 ng et 100 ng. L'intégration de l'aire des pics correspondant à l'OCDO pour ces différentes quantités d'OCDO ont permis d'établir une courbe de calibration permettant de quantifier l'OCDO . Cette méthode permet de doser des quantités d'OCDO comprises entre 5 et 200 ng (voir figure 17).

### Exemple 5 : Inhibition de la production d'OCDO par le tamoxifène in vivo.

On a déjà montré à l'essai 2 (figure 3A) que l'addition de tamoxifène sur les cultures de cellules tumorales MCF7 bloque la production d'OCDO dans ces cellules. On va montrer ci-après que le tamoxifène administré in vivo entraîne aussi une inhibition de la production d'OCDO.

On a utilisé les techniques de dosage détaillées à l'exemple 4 pour mesurer la modulation de l'OCDO sur des tumeurs implantées chez la souris.

On effectue d'abord une culture cellulaire de cellules TS/A. Ces cellules TS/A sont des adénocarcinomes mammaires de souris (voir Nanni P et al., Clin. Exp. Metastasis, 1983 Oct-Dec ; 1(4):373-80). Les cellules sont cultivées, à 37°C sous 5% CO2, dans un milieu DMEM supplémenté par 2g/litre de carbonate de sodium, 1,2 mM de glutamine (pH 7,4 à 23°C), 5% de sérum bovin foetal (Gibco) et 2,5 ml d'antibiotiques par litre de milieu (pénicilline/streptomycine).

Les cellules TS/A sont récupérées avec de la trypsine, lavées deux fois et suspendues dans du tampon PBS. Les cellules TS/A (environ 4 10⁶ 15 cellules /0.1 ml) sont ensuite injectées en sous-cutané dans le flanc de souris femelles BALB/c de 6 semaines (fournies par Charles River). Les animaux sont traités 27 jours après l'implantation des tumeurs, par voie intratumorale une fois par jour sur une durée de 37 jours, soit avec du tamoxifène à une concentration de 10 µM pour des volumes d'injection de 100 µl (groupe traité par le tamoxifène) soit avec le solvant véhicule (éthanol à 0,1 % dans du tampon phosphate PBS (groupe contrôle). Les animaux (10 souris par groupe) sont suivis régulièrement pour le développement des tumeurs. Le volume des tumeurs est calculé selon la formule suivante Lx1²x0.5 (L est la longueur et 1 est la largeur de la tumeur). L'expérience a été reproduite deux fois. Les résultats sont fournis sur la figure 18 (la flèche indique le début du traitement).

On observe donc qualitativement, sur la figure 18, une inhibition de la pousse tumorale chez les souris traitées au tamoxifène.

Pour obtenir une information quantitative sur l'inhibition de l'OCDO, l'expérience a été reproduite dans les mêmes conditions et les tumeurs ont été prélevées au jour 28. On ajoute 5 volumes (par rapport à la masse prélevée) de tampon (Tris-HCl 50 mM, KCl 150 mM, pH=7,4). Les tumeurs sont broyées et les homogénats sont centrifugés pendant 5 minutes à 2500 tours/mn à 4°C. On récupère les surnageants puis on ajoute 1 volume de méthanol (par rapport au volume de surnageant) et 2 volumes de chloroforme. Après centrifugation (pour séparer les phases), on récupère la phase organique. La phase organique est évaporée à sec puis le résidu est repris par 0.5 ml de chloroforme. Les extraits organiques sont filtrés sur des cartouches de silice de 0.5 ml. Les stérols polaires sont élués de façon séquentielle par :
1) 0.5 ml d'un mélange hexane/chloroforme (1/1)
2) 0.5 ml de chloroforme,
3) 0.5 ml d'acétate d'éthyle et du méthanol.

L'addition d'un standard externe OCDO marqué au ¹⁴C durant les phases d'extraction et de purification permet d'établir un rendement de récupération de 86±6%. Les fractions acétate d'éthyle sont évaporées, sillylées (50 µl acétonitrile 1/BSTFA 1) puis analysées par GC/MS (2µl) selon les conditions décrites précédemment.

La figure 19 présente deux chromatogrammes faits en phase gazeuse comme indiqué à l'exemple 4, correspondant :
A) à un extrait de tumeur provenant d'un animal témoin : la tumeur avait une masse de 2 g ;
B) à un extrait de tumeur provenant d'un animal traité au tamoxifène : la tumeur avait une masse de 1,45 g.

On observe sur la figure 19 une diminution significative des pics provenant de l'OCDO dans la tumeur des souris traitées (B) par comparaison à celle des souris non traitées (A). L'analyse en spectrométrie de masse confirme la structure des molécules dans les pics d'intérêt (voir figure 20).

On a donné ci-dessous les résultats chiffrés ainsi obtenus, le groupe témoin ayant été précédemment défini, et on a valorisé à 100 les valeurs associées au témoin par des coefficients qui ont été également appliqués aux résultats relatifs au tamoxifène.

| | **Jour 28** | | **Jour 37** | |
|---|---|---|---|---|
| **molécule** | **taille de la tumeur** (% du contrôle) | **OCDO** (% contrôle) | **taille de la tumeur** (% du contrôle) | **OCDO** (% contrôle) |
| **Témoin non traité** | 100 | 100 | 100 | 100 |
| **Tamoxifène** | 73,5±8 | 19,2±4 | 46,2±7 | 8,4±4 |

On voit que, in vivo, le tamoxifène inhibe significativement l'OCDO.

Le fait que le tamoxifène exerce une activité anti tumorale tout en inhibant in vivo la production d'un oxystérol promoteur de tumeur montre que l'OCDO joue un rôle dans les effets du tamoxifène.

### Exemple 6 : Inhibition de la production d'OCDO par le PBPE in vivo.

Le PBPE est un composé dont l'effet antiprolifératif a été établi par des expériences in vitro (voir la référence citée à l'essai 2 et aussi la publication Payre B. et al, Mol. Cancer Ther., 7(12), 3707-3717). On a déterminé, pour le PBPE, des résultats in vivo analogues à ceux présentés dans l'exemple 5 pour le tamoxifène. Le protocole utilisé est strictement identique à celui qui a été détaillé à l'exemple 5 pour le traitement au tamoxifène à cette différence près que les injections intratumorales journalières sont effectuées à une concentration de 40 µM pour le PBPE pour des volumes d'injection de 100 µL. Les résultats sont regroupés dans le tableau 5 ci-dessous :

| | **Jour 28** | | **Jour 37** | |
|---|---|---|---|---|
| **molécule** | **taille de la tumeur** (% du contrôle) | **OCDO** (% contrôle) | **taille de la tumeur** (% du contrôle) | **OCDO** (% contrôle) |
| **Témoin non traité** | 100 | 100 | 100 | 100 |
| **PBPE** | 71,6±7 | 22,5±5 | 54,5±8 | 9,6±5 |

On a donc déterminé in vivo que le PBPE d'une part inhibe l'OCDO et d'autre part, fait régresser les tumeurs.

**Exemple 7 :** Inhibition de la production d'OCDO par PBPE sur diverses cellules tumorales. L'essai 2 précédemment fourni a montré que des extraits cellulaires de cellules tumorales MCF7 incubées pendant 3 jours dans des solutions de PBPE conduisent à constater une inhibition du métabolite que l'on a identifié comme étant l'OCDO.

On a repris une expérience analogue avec des lignées différentes en incubant les cellules testées pendant 48 heures dans une solution à 40 µM de PBPE. On a constaté que l'OCDO était inhibé à 100% pour toutes les lignées testées du tableau suivant :

| **Lignée cellulaire** | **Type** | **Inhibition de la production d'OCDO** |
|---|---|---|
| MCF-7 | Carcinome mammaire Humain | 100 % |
| MDA-MB-231 | Carcinome mammaire Humain | 100% |
| A-549 | Carcinome pulmonaire Humain | 100% |
| B-16-F10 | Mélanome murin | 100% |
| U-937 | Leucémie Humaine | 100% |
| HT-29 | Carcinome colique Humain | 100% |
| HeLa | Carcinome utérin Humain | 100 % |
| C6 | Gliome de rat | 100 % |
| SH-N-SH | Neuroblastome Humain | 100% |
| Saos-2 | Osteosarcome Humain | 100 % |
| P19 | Lignée carcinoembryonaire murine | 100 % |
| TT | Carcinome médulaire de la thyroïde Humain | 100 % |

De façon générale, pour établir les pourcentages d'inhibition de l'OCDO à partir des plaques de chromatographie en couche mince (CCM) obtenues comme à l'essai 2, les métabolites radioactifs sont identifiés et quantifiés à partir desdites plaques en utilisant une plaque sensible à l'europium de type « GP Phosphorescreen » (GE Healthcare) et un « Phosphor Imagor Storm 840 » (GE Healthscare). La proportion des oxystérols radio-marqués est déterminée sur l'autoradiogramme obtenu par densitométrie en utilisant le logiciel « Image 5 Quant 5.2 ». Le pourcentage est calculé à partir du rapport entre la quantité d'oxystérols quantifiés divisée par la somme des quantités d'oxystérols (CEE + CE + CT + OCDO). Le CEE (ester de CE) provenant exclusivement du CE, le pourcentage du CE est calculé à partir du rapport (CE + CEE)/(CE + CEE + CT + OCDO).

### Exemple 8 : L'OCDO est inhibé quand la ChEH est inactive.

Comme il a été établi précédemment dans cette demande de brevet, on sait que les cellules tumorales produisent de l'OCDO, ce qui implique que l'hydrolase ChEH est active puisqu'elle permet la transformation de CE en CT qui est le passage obligé pour la production d'OCDO. Comme il sera établi dans le tableau 1 donné plus loin dans cet exemple, le tamoxifène, le PBPE, le raloxifène et le DPPE sont des inhibiteurs de la ChEH. On a donc complété l'essai 2 précédemment fourni en mesurant les quantités des produits en présence lorsque les cellules tumorales MCF7 sont incubées comme indiqué en détail dans l'essai 2. Le calcul des pourcentages de CE, CT et OCDO a été effectué comme indiqué à l'exemple 7. Les résultats sont fournis à la figure 21, qui montre une plaque de chromatographie en couche mince sur laquelle les pistes correspondant à des incubations des cellules avec le solvant véhicule T (0,1% éthanol dans du tampon PBS), avec CE-_ et avec quatre inhibiteurs de ChEH dont les identifiants sont donnés en bas des pistes. Les valeurs numériques obtenues sont rassemblées dans le tableau ci-dessous :

| Composés | % CE | % OCDO | % CT |
|---|---|---|---|
| EtOH (T) | 0 | 73,1 | 26,9 |
| CE α 10 µM | 31,5 | 27 | 41,5 |
| Tam 2,5 µM | 90,3 | 6,9 | 2,8 |
| PBPE 10 µM | 89,2 | 6,2 | 4,6 |
| Ral 10 µM | 96,5 | 0,5 | 3 |
| DPPE 1,5 µM | 80,7 | 8,8 | 10,5 |
| T = témoin | | | |
| DPPE = N,N-Diethyl-2-(4-benzylphénoxy)éthanamine | | | |
| Ral = Raloxifene | | | |

On constate qu'une inhibition de ChEH entraine bien une inhibition de l'OCDO, le produit CT étant alors en quantité 5 très réduite.

En fait, la formation de l'OCDO dans une cellule dépend de plusieurs paramètres :
1. la présence de cholestérol
2. la présence de CE
3. la présence de cholestérol époxyde hydrolase (ChEH)
4. la présence de CT
5. le transport du CT vers la zone d'oxydation du CT en OCDO
6. la présence et l'activité de l'enzyme responsable de l'oxydation de CT en OCDO Pour établir le bien fondé du lien existant entre la capacité de divers composés d'inhiber l'OCDO dans une cellule tumorale MCF7, d'une part, et leur qualité d'inhibiteur de la ChEH, d'autre part, on a calculé les coefficients d'inhibition Ki pour ChEH relatifs à un certain nombre de produits et on a mesuré la production d'OCDO par les MCF7 après incubation des cellules par les mêmes produits selon le protocole défini dans l'essai 2 de la présente demande de brevet. L'ensemble des résultats est présenté dans les tableaux 1 et 2. A partir de ces deux tableaux, il apparaît que tout inhibiteur d'enzyme impliqué dans la biosynthèse de cholestérol entraine une diminution du cholestérol utilisable pour la production de l'OCDO. On notera, en particulier, des inhibiteurs des enzymes à partir de l'hydroxyméthylglutaryle Coenzyme A synthétase (HMG ÇoA synthétase) jusqu'à la 7-déhydrocholestérol réductase et la 24-déshydrocholestérol réductase.

Une expérience a été effectuée dans cette catégorie d'inhibiteurs, à savoir, avec un inhibiteur d'HMG-coA réductase, tel que la « Lovastatine », utilisé à une concentration de 10µM : on a constaté qu'après une incubation de cellules MCF7 selon le protocole donné dans l'essai 2 et en amenant le témoin à la valeur 100, on obtient une production d'OCDO par les MCF7 de moins de 1.

**TABLEAU 1**

| | **Molécule** | ***Kᵢ* ChEH (nM)** |
|---|---|---|
| **ligands d'AEBS** | PBPE | 26,8 ± 6 |
| | PCPE | 34,7 ± 8 |
| | Tesmilifene | 62,4 ± 3 |
| | DDA | 1250,4 ± 6 |
| **modulateurs des récepteurs des oestrogènes** | Tamoxifène | 33,6 ± 8 |
| | 4OH-Tamoxifène | 145,3 ± 4 |
| | Raloxifene | 35,6 ± 4 |
| | Nitromiphene | 17,7 ± 6 |
| | Clomiphene | 9,0 ± 2 |
| | RU 39,411 | 155,2 ± 8 |
| **ligands des récepteurs sigma** | BD-1008 | 98,7 ± 9 |
| | Haloperidol | 18067 ± 14 |
| | SR-31747A | 6,2 ± 2 |
| | Ibogaine | 2150 ± 11 |
| | AC-915 | 3527 ± 9 |
| | Rimcazole | 2325 ± 8 |
| | Amiodarone | 733,1 ± 9 |
| | Trifluoroperazine | 135,4 ± 7 |
| **inhibiteurs de la biosynthèse du Cholestérol** | Ro 48-8071 | 88,9 ± 5 |
| | U-18666A | 90,3 ± 5 |
| | AY-9944 | 649 ± 6 |
| | Triparanol | 39,5 ± 3 |
| | Terbinafine | 9105 ± 33 |
| | SKF-525A | 1904 ± 11 |

**TABLEAU 2**

| | **Molécule** (concentration en µM) | **Production d'OCDO par les MCF-7** (%contrôle) |
|---|---|---|
| **Témoin** | - | 100 |
| **ligands d'AEBS** | PBPE (1) | <1 |
| | PCPE (1) | <1 |
| | Tesmilifene (1) | <1 |
| | DDA | <1 |
| **modulateurs des récepteurs des oestrogènes** | Tamoxifène (1) | <1 |
| | 40H-Tamoxifène (1) | <1 |
| | Raloxifene (1) | <1 |
| | Nitromiphene (1) | <1 |
| | Clomiphene (1) | <1 |
| | RU 39,411 (5) | <1 |
| **ligands des récepteurs σ** | BD-1008 (1) | <1 |
| | Haloperidol (100) | 42 |
| | SR-31747A (1) | <1 |
| | Ibogaine (5) | 25 |
| | AC-915 (10) | 16 |
| | Rimcazole (5) | 12 |
| | Amiodarone (5) | 8 |
| | Trifluoroperazine (1) | <1 |
| **inhibiteurs de la biosynthèse du Cholestérol** | Ro 48-8071 (10) | <1 |
| | U-18666A (1) | <1 |
| | AY-9944 (5) | <1 |
| | Triparanol (1) | <1 |
| | Terbinafine (10) | <1 |
| | SKF-525A (10) | <1 |

Les produits qui apparaissent dans les tableaux 1 et 2 et qui n'ont pas été précédemment identifiés dans le présent texte sont définis par leur nom chimique dans la liste suivante :
PCPE: 1-(2-(4-(2-phenylpropan-2-yl)phenoxy)ethyl)pyrrolidine;
tesmilifene: 2-(4-benzylphenoxy)-N,N-diethylethanamine,.

tamoxifène: 2-[4-[(Z)-1,2-di(phenyl)but-1-enyl]phenoxy]-N,N-dimethylethanamine;
40H-tamoxifène: 4-[(Z)-1-[4-(2-dimethylaminoethyloxy)phenyl]-2-phenylbut-1-enyl]phenol;
raloxifene: [6-hydroxy-2-(4-hydroxyphenyl)-1-benzothiophen-3-yl]-[4-(2-piperidin-1-ylethoxy) phenyl] methanone;
nitromiphene: 1-[2-[4-[(Z)-1-(4-methoxyphenyl)-2-nitro-2-phenylethenyl]phenoxy] ethyl]pyrrolidine;
clomiphene: 2-[4-[(Z)-2-chloro-1,2-di(phenyl)ethenyl]phenoxy]-N,N-diethylethanamine;
RU 39411: 11-[4-*N,N*-[diethylaminoethoxy]phenyl]-estra-1,3,5(10) triene-3,17-diol.

BD-1008: N-(3,4-dichlorophenethyl)-N-methyl-2-(pyrrolidin-1-yl)ethanamine;
Haloperidol : 4-(4-(4-chlorophenyl)-4-hydroxypiperidin-1-yl)-1-(4-fluorophenyl)butan-1-one; SR-31747A: (E)-N-(4-(3-chloro-4-cyclohexylphenyl)but-3-enyl)-N-ethylcyclohexanamine;
AC915: N-(3,4-dichlorophenethyl)-N-methyl-2-(pyrrolidin-1-yl)ethanamine
Rimcazole: 9-[3-[(3S,5R)-3,5-dimethylpiperazin-1-yl]propyl]carbazole
Amiodarone: (2-butyl-1-benzofuran-3-yl)-[4-(2-diethylaminoethyloxy)-3, 5-diiodophenyl]methanone
Trifluoroperazine: 10-[3-(4-methylpiperazin-1-yl)propyl]-2-(trifluoromethyl)phenothiazine

RO 48-8071: (4-(6-(allyl(methyl)amino)hexyloxy)phenyl)(4-bromophenyl)methanone;
U-18666A: 3-beta-(2-(diethylamino)ethoxy)androst-5-en-17-one;

AY-9944: trans-1,4-Bis(2-chlorobenzaminomethyl)cyclohexane;
Triparanol : 2-(4-chlorophenyl)-1-(4-(2-(diethylamino)ethoxy)phenyl)-1-p-tolylethanol;
terbinafine: (E)-N,3,6,6-tetramethyl-N-(naphthalene-1-ylmethyl)hept-2-en-4-yn-1-amine; SKF-525A: 2-diethylaminoethyl2,2-diphenylpen tanoate.

Les inhibiteurs, qui ont fait l'objet des expériences ayant abouties aux tableaux 1 et 2 incluent des modulateurs des récepteurs des oestrogènes, des ligants des sites de liaison membranaires des anti-oestrogènes (AEBS), des ligants des récepteurs sigma 1 et 2, des inhibiteurs de la biosynthèse du cholestérol à partir de la squalène synthétase et jusqu'à la 7- et 24-déshydrocholestérol réductase. Tous ces inhibiteurs ont un point commun, à savoir, celui d'être des ligands des sites AEBS.

On a détaillé ci-après les protocoles utilisés pour la mesure des coefficients d'inhibition Ki du Tableau 1 :

### a) Ki pour les AEBS

Le Ki est la constante d'inhibition correspondant à la molécule d'intérêt et est mesuré de la façon suivante : des microsomes de foie de rat sont incubés avec une concentration de 2,5nM de tamoxifène tritié (fourni par la société GE healthcare) et des concentrations croissantes en inhibiteur comprises entre 0,01 et 1000µM dans les conditions décrites dans la publication suivante : Poirot M. et al, Bioorg Med Chem 2000, vol 8(8), p. 2007-2016. Les IC50 correspondent à la concentration en inhibiteur requise pour inhiber 50% de l'activité de la ChEH ; E elles sont déterminées par utilisation d'un programme de traitement de données « GraphPad Prizm (version 4) ». Les Ki sont calculés par utilisation de l'équation de Cheng et Prussof (Cheng et Prussolf, Biochem Pharmacol, 1973, vol 22(23), pages 3099- 3108). Le Ki est exprimé selon l'équation : Ki = [IC50](1+([tamoxifène tritié])/Kd). La concentration en tamoxifène tritié est de 2,5 nM et la constante de dissociation a l'équilibre du tamoxifène tritié pour AEBS est de 2 nM.

### b) Détermination du Ki pour ChEH :

150 µg de protéines de microsomes de foie de rat sont incubés en présence de 2 concentrations de CE-α[¹⁴C] avec des concentrations croissantes d'inhibiteurs comprises entre 0,01 et 1000 µM dans les conditions décrites précédemment pour la mesure de l'activité ChEH. Le Ki est mesuré comme la projection sur l'axe des abscisses de l'intersection des lignes obtenues en reportant sur un graphe les valeurs de 1/V en fonction de 1/S pour la ChEH, tel que déterminé par la méthode de Dixon (Dixon M, Biocheml Jl, 1953, vol 55(1), p 170-171).

### Exemple 9 : Inhibition de l'OCDO par un inhibiteur des cytochromes P450.

L'époxydation du cholestérol peut être produite par auto-oxydation du cholestérol avec de l'oxygène de l'air, sous l'action d'enzymes comme des cytochromes P450 ou des lipoxygénases (voir Schroepfer G.Jr, Physiological Reviews, Vol. 80, n° 1, p. 361-554, 2000).

On a constaté une inhibition de la production de l'époxystérol CE et de ses dérivés, le CT et l'OCDO, par utilisation d'un inhibiteur général des cytochromes P450, à savoir, le ketoconazole (voir figure 22).

Le protocole pour cette expérience est le même que celui décrit dans l'exemple 7.

### Exemple 10 : Inhibition de la ChEH par un aminoalkylstérol.

Pour cette expérience, on a choisi un aminoalkylstérol inclus dans le brevet français 2 838 741, (et aussi voir : de Médina et al, J Med Chem :2009 Vol 52, n°23, p. 7765-7777) ayant comme formule : Cholestan-3β,5α-diol6β-N-[2-(3H-imidazole-4-yl)-éthylamino] (DDA). On a incubé des cellules tumorales MCF7 par du CE[¹⁴C](10mCurie/mmol, 0,6µM) pendant 48 heures en présence ou en l'absence de l'aminoalkylstérol susmentionné (aux concentrations de 0,1 et 1 µM).

On a effectué une chromatographie en couche mince, dont la plaque a été reproduite sur la figure 23. Sur cette plaque, l'incubation est faite pour la piste 1 avec CE[¹⁴C] ; pour la piste 2, avec CT[¹⁴C] ; pour la piste 3, avec le solvant véhicule, qui est le même que celui utilisé pour les essais 1 à 5 ; pour la piste 4, avec CE[¹⁴C]et 0,1 µM de l'aminoalkylstérol ; et pour la piste 5, avec CE[¹⁴C] et une concentration de 1 µM de l'aminoalkylstérol.

On observe que la présence de l'aminoalkylstérol entraine une inhibition de la ChEH ; cette inhibition est dépendante de la concentration en aminoalkylstérol.

Par ailleurs, on a également effectué une étude de cette inhibition sur homogénat. Le protocole est le suivant : les cellules MCF7 sont décrochées à la trypsine et reprises par du milieu RPMI 5%FBS. La suspension cellulaire obtenue (60 millions de cellules) est centrifugée, lavée au PBS froid et resuspendue dans un 1ml de tampon Tris-HCL 20 (pH=7,4 ; 150 mM KCL). Les cellules sont lysées par cinq cycles de congélation/décongélation (azote liquide/37°C). La solution est centrifugée à 1200 tours/mn à 4°C pendant 10 minutes. Le surnageant est récupéré et la quantité de protéines est déterminée par la méthode de Bradford. La mesure de l'activité ChEH sur lysat cellulaire MCF7 est 25 effectuée comme suit : l'activité enzymatique est mesurée sur 150 µg de protéines dans un volume final de 150 µl comportant 125 µl de tampon ChEH (trisHCL, pH 7,4, 150mM KCL) et 15 µl de protéines MCF7. On a comparé les valeurs des IC 50 et on a constaté que (IC 50)cellules = 0,6µM alors que (IC50)_{homogénat}=11,2 µM.

Cette différence entre les IC50 montre que l'aminoalkylstérol testé présente des propriétés de prévention de la survenue des cancers.

On a, en outre, déterminé, que pour le composé DDA, des résultats analogues à ceux présentés dans l'exemple 6 pour le PBPE. Le protocole utilisé est strictement identique à celui qui a été détaillé à l'exemple 5 pour le traitement au tamoxifène à cette différence près que les injections intro-tumorales journalières sont effectuée à une concentration de 10 µM pour des volumes d'injection de 100 µL. Les résultats sont regroupés dans le tableau ci-dessous :

| | **Jour 28** | | **Jour 37** | |
|---|---|---|---|---|
| **molécule** | **taille de la tumeur** (% du contrôle) | **OCDO** (% contrôle) | **taille de la tumeur** (% du contrôle) | **OCDO** (% contrôle) |
| **Témoin non traité** | 100 | 100 | 100 | 100 |
| **DDA** | 65,4±7 | 14,5±3 | 28,3±8 | 4,5±3 |

On a donc établi, dans cet exemple, que d'une part, le produit DDA inhibe l'OCDO et que, d'autre part, in vivo, il réduit la taille tumorale.

### Exemple 11 : Inhibition de l'OCDO par des modulateurs du transport intracellulaire du cholestérol et des modulateurs des récepteurs arylhydrocarbure (récepteur Ahr).

On a testé deux modulateurs du transport intracellulaire du cholestérol, à savoir, la progestérone et le « U18666A » (3-β-(2-20 (diéthylamino)ethoxy)androst-5-en-17-one) (voir LISCUM L et al,. J. Biol. Chem., vol 270 (26) p 15443-15446, 1995) (pistes 2 et 3 respectivement).

On a testé également (voir pistes 4 et 5 respectivement) deux modulateurs des récepteurs Ahr (Aryl hydrocarbon receptor), à savoir, la 2,3,7,8-tétrachlorodibenzo-p-dioxine (TCDD) et le Benzo(A)pyrène. On a enfin testé deux antagonistes des récepteurs Ahr, à savoir, le resvératrol et le 1,3-dichloro-5-[(1E)-2-(4-chlorophényl)ethényl]-5 benzène (PDM2) (voir : « Casper R.F. et al, Mol. Pharmacol., 1999 oct. ; 56(4) ; 784-90 » et « de Médina et al, J.Med Chem., 2005 Janv. 13 ;48(1) : 287-91) »).

On a réalisé les expériences suivantes : des cellules tumorales MCF7 ont été mises en présence de 0,5 µM de CT [¹⁴C] et ont été ensuite incubées pendant 24 heures en présence de l'un des produits suivants :
1. solvant véhicule (0,1% d'éthanol dans un tampon PBS) servant de témoin (piste 1)
2. 10 µM de progestérone
3. 10 µM de U18666A
4. 100 nM de TCDD
5. 10 µM de benzo(A)pyrène
6. 10 µM de resvératrol
7. 10 µM de PDM2 (Ant. 1)

On a réalisé des chromatographies sur couche mince et la figure 24 représente la plaque obtenue. A partir des taches des différentes pistes, on a quantifié, en appliquant le protocole détaillé dans l'exemple 7, la production de l'OCDO dans les cellules traitées. Les résultats de cette quantification sont représentés par l'histogramme de la figure 24.

On a, en outre, mesuré les quantités d'OCDO produites par des cellules 25 MCF7, quand on les incube selon le même protocole que ci-dessus défini avec d'autres antagonistes des récepteurs Ahr, le test témoin étant amené à 100 et la production d'OCDO étant exprimée en % du témoin (même témoin que ci-dessus). Les résultats sont donnés dans le tableau suivant :

| | **Molécule** (concentration 10 µM) | **Production d'OCDO par les MCF-7** (%contrôle) |
|---|---|---|
| **Témoin** | - | 100 |
| **Antagoniste des récepteurs Ahr** | Resveratrol | <1 |
| | Ant 1(10) | <1 |
| | Ant 2(10) | <1 |
| | Ant 3(10) | <1 |
| | Ant 4(10) | <1 |
| | Ant 5(10) | <1 |
| Ant. 1: (E)-1-(4'-cholorophenyl)-2-(3,5-dichlorophenyl)-ethene; | | |
| Ant. 2: (E)-1-(4'-methoxyphenyl)-2-(3,5-fluorophenyl)-ethene; | | |
| Ant. 3: (E)-1-(4'-fluorophenyl)-2-(3,5-fluorophenyl)-ethene; | | |
| Ant. 4: (E)-1-(4'-triluoromethyl-phenyl)-2-(3,5-rifluoromethyl-phenyl)-ethene; | | |
| Ant. 5: (E)-1-(4'-fluorophenyl)-2-(3,5-dimethoxyphenyl)-ethene. | | |

Il apparaît donc que les modulateurs du transport intro-5 cellulaire du cholestérol et les antagonistes des récepteurs Ahr peuvent inhiber la formation de l'OCDO et être utilisés en conséquence pour leur effet anticancéreux.

## Revendications

1. Méthode pour détecter l'état oncogénique de cellules d'un échantillon obtenu chez un sujet, comprenant une étape où l'on détermine la quantité du composé OCDO de formule 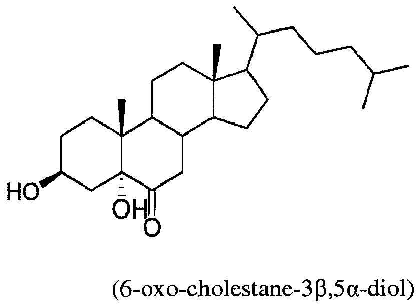 présente dans lesdites cellules.

2. Méthode pour diagnostiquer un cancer chez un sujet, comprenant une étape de détection de l'état oncogénique de cellules d'un échantillon obtenu chez un sujet selon la méthode de la revendication 1
dans laquelle une quantité d'OCDO supérieure à une valeur de référence mesurée dans les cellules d'un échantillon de sujet sain est indicative d'un état oncogénique des cellules dudit échantillon.

3. Méthode pour suivre la réponse à un traitement d'un sujet souffrant d'un cancer, ladite méthode comprenant les étapes de détection, avant et au cours du traitement, de l'état oncogénique de cellules d'un échantillon obtenu chez ledit sujet selon la méthode de la revendication 1;
un changement de l'état oncogénique du sujet au cours du traitement étant indicatif d'une réponse du sujet audit traitement.

4. Méthode pour évaluer l'efficacité d'un médicament pour traiter un cancer chez un sujet atteint par ledit cancer, comprenant:
(a) une étape de détection de l'état oncogénique de cellules d'un échantillon obtenu chez un sujet selon la méthode de la revendication 1 où on dose la concentration D1 d'OCDO dans un extrait liquide des cellules d'un échantillon obtenu chez ledit sujet;
(b) après un temps de traitement thérapeutique, une étape de détection de l'état oncogénique de cellules d'un échantillon obtenu chez un sujet selon la méthode de la revendication 1 où on dose la concentration D2 d'OCDO dans un extrait liquide des cellules d'un échantillon obtenu chez ledit sujet de la même manière qu'à l'étape (a);
(c) une étape de comparaison de D1 et D2 ; et
(d) si D2 < D1, on en déduit que le médicament est efficace pour traiter ledit cancer.

5. Méthode pour évaluer l'efficacité d'un traitement du cancer chez un sujet atteint par ledit cancer, ladite méthode comprenant les étapes de détection, avant et au cours du traitement, de l'état oncogénique de cellules d'un échantillon obtenu chez ledit sujet selon la méthode de la revendication 1; un changement de l'état oncogénique ou du diagnostic du sujet au cours du traitement étant indicatif de l'efficacité dudit traitement pour traiter le cancer.

6. Méthode selon l'une des revendications 1 à 5 dans laquelle l'échantillon est un échantillon de sang.

7. Méthode selon l'une des revendications 1 à 5 dans laquelle l'échantillon est une biopsie.

8. Méthode selon la revendication 1 ou 2 dans laquelle l'échantillon est une biopsie d'un tissu suspecté d'être cancéreux.

9. Méthode selon la revendication 1, 3, 4 ou 5 dans laquelle l'échantillon est une biopsie d'un tissu cancéreux.

10. Utilisation du composé OCDO présent dans des cellules d'un échantillon obtenu chez un sujet comme marqueur de l'état oncogénique desdites cellules.

11. Inhibiteur de l'OCDO destiné à être utilisé dans une méthode de traitement d'un cancer chez l'homme ou l'animal, **caractérisé en ce qu'**il est choisi parmi :
- les inhibiteurs de l'activité cholestérol-époxy hydrolase (ChEH) choisis dans le groupe consistant en le Ro 48-8071 et l'ibogaïne.

## Patentansprüche

1. Verfahren zum Detektieren des onkogenen Zustands von Zellen einer Probe, die von einem Subjekt erhalten wurde, umfassend einen Schritt, in dem die Menge der Verbindung OCDO der Formel 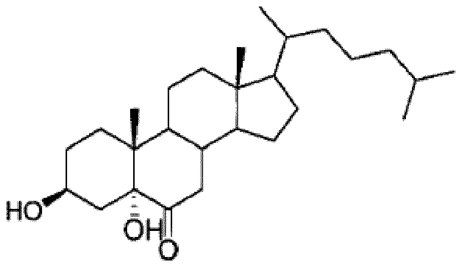 (6-Oxo-cholestan-3β,5α-diol),
die in den Zellen vorhanden ist, bestimmt wird.

2. Verfahren zum Diagnostizieren einer Krebserkrankung in einem Subjekt, umfassend einen Detektionsschritt des onkogenen Zustands von Zellen einer Probe, die von einem Subjekt erhalten wurde, gemäß dem Verfahren von Anspruch 1,
wobei eine Menge von OCDO, die größer ist als ein Referenzwert, der in den Zellen einer Probe eines gesunden Subjekts gemessen wurde, indikativ für einen onkogenen Zustand der Zellen der Probe ist.

3. Verfahren zum Verfolgen des Ansprechens auf eine Behandlung eines Subjekts, das an einer Krebserkrankung leidet, wobei das Verfahren die Detektionsschritte, vor und während der Behandlung, des onkogenen Zustands von Zellen einer Probe, die von dem Subjekt erhalten wurde, gemäß dem Verfahren von Anspruch 1 umfasst;
wobei eine Änderung des onkogenen Zustands des Subjekts während der Behandlung indikativ für ein Ansprechen des Subjekts auf die Behandlung ist.

4. Verfahren zum Beurteilen der Wirksamkeit eines Medikaments zum Behandeln einer Krebserkrankung in einem Subjekt, das an der Krebserkrankung erkrankt ist, umfassend:
(a) einen Detektionsschritt des onkogenen Zustands von Zellen einer Probe, die von einem Subjekt erhalten wurde, gemäß dem Verfahren von Anspruch 1, wobei die Konzentration D1 von OCDO in einem flüssigen Extrakt der Zellen einer Probe, welche von dem Subjekt erhalten wurde, bestimmt wird;
(b) nach einem therapeutischen Behandlungszeitraum, einen Detektionsschritt des onkogenen Zustands von Zellen einer Probe, die von einem Subjekt erhalten wurde, gemäß dem Verfahren von Anspruch 1, wobei die Konzentration D2 von OCDO in einem flüssigen Extrakt der Zellen einer Probe, welche von dem Subjekt erhalten wurde, bestimmt wird, auf die gleiche Weise wie in Schritt (a);
(c) einen Vergleichsschritt von D1 und D2; und
(d) wenn D2 < D1, wird daraus geschlussfolgert, dass das Medikament wirksam zum Behandeln der Krebserkrankung ist.

5. Verfahren zum Beurteilen der Wirksamkeit einer Behandlung einer Krebserkrankung in einem Subjekt, das an der Krebserkrankung erkrankt ist, wobei das Verfahren die Detektionsschritte, vor und während der Behandlung, des onkogenen Zustands von Zellen einer Probe, die von dem Subjekt erhalten wurde, gemäß dem Verfahren von Anspruch 1 umfasst; wobei eine Änderung des onkogenen Zustands oder der Diagnose des Subjekts während der Behandlung indikativ für eine Wirksamkeit der Behandlung zum Behandeln der Krebserkrankung ist.

6. Verfahren nach einem der Ansprüche 1 bis 5, wobei die Probe eine Blutprobe ist.

7. Verfahren nach einem der Ansprüche 1 bis 5, wobei die Probe eine Biopsie ist.

8. Verfahren nach Anspruch 1 oder 2, wobei die Probe eine Biopsie eines Gewebes ist, welches vermutet wird krebskrank zu sein.

9. Verfahren nach Anspruch 1, 3, 4 oder 5, wobei die Probe eine Biopsie eines krebskranken Gewebes ist.

10. Verwendung der Verbindung OCDO, die in Zellen einer Probe, welche von einem Subjekt erhalten wurde, vorhanden ist, als Marker für den onkogenen Zustand der Zellen.

11. Inhibitor von OCDO, vorgesehen zur Verwendung in einem Behandlungsverfahren einer Krebserkrankung in einem Mensch oder in einem Tier, **dadurch gekennzeichnet, dass** er ausgewählt ist aus:
- Inhibitoren der Cholesterol-Epoxid-Hydrolase (ChEH)-Aktivität, ausgewählt aus der Gruppe, bestehend aus Ro 48-8071 und Ibogain.

## Claims

1. A method for detecting the oncogenic state of cells of a sample obtained from an individual, comprising a step in which the amount of the OCDO compound of formula 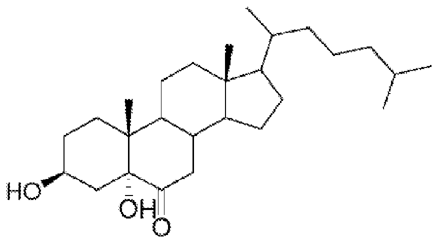 (6-oxo-cholestane-3β,5α-diol)
present in said cells is determined.

2. A method for diagnosing cancer in an individual, comprising a step of detection of the oncogenic state of cells of a sample obtained from an individual according to the method of claim 1
wherein an amount of OCDO greater than a a reference value measured in the cells of a sample of a healthy individual is indicative of an oncogenic state of cells of said sample.

3. A method for monitoring the response to a treatment of an individual suffering from cancer, said method comprising the steps of detection, before and during the treatment, of the oncogenic state of cells of a sample obtained from said individual according to the method of claim 1;
a change in the oncogenic state of the individual during the treatment being indicative of a response by the individual to said treatment.

4. A method for evaluating the efficacy of a medicament for treating a cancer in an individual suffering from said cancer, comprising
(a) a step of detection of the oncogenic state of cells of a sample obtained from an individual according to the method of claim 1 wherein the concentration D1 of OCDO in a liquid extract of the cells of a sample obtained from said individual is assayed;
(b) after a therapeutic treatment time, a step of detection of the oncogenic state of cells of a sample obtained from an individual according to the method of claim 1 wherein the concentration D2 of OCDO in a liquid extract of the cells of a sample obtained from said individual is assayed in the same way as in step (a);
(c) a step of comparing D1 and D2; and
(d) if D2 < D1, it is deduced therefrom that the medicament is effective for treating said cancer.

5. A method for evaluating the efficacy of a cancer treatment in an individual suffering from said cancer, said method comprising the steps of detection, before and during the treatment, of the oncogenic state of cells of a sample obtained from said individual according the method of claim 1; a change in the oncogenic state or in the diagnosis of the individual during the treatment being indicative of the efficacy of said treatment for treating the cancer.

6. The method according to any of claims 1 to 5 wherein the sample is a blood sample.

7. The method according any of claims 1 to 5 wherein the sample is a biopsy.

8. The method according to claim 1 or 2 wherein the sample is a biopsy from a tissue suspected to be cancerous.

9. The method according to claim 1, 3, 4 or 5 wherein the sample is a biopsy from a cancerous tissue.

10. Use of an OCDO compound present in cells of a sample obtained from an individual as a marker of oncogenic state of said cells.

11. An OCDO inhibitor for use in a method for treating cancer in humans or animals **characterized in that** it is selected among:
- inhibitors of cholesterol epoxide hydrolase (ChEH) activity selected from the group consisting of Ro 48-8071 and ibogaine.
